(19) **Europäisches Patentamt** / **European Patent Office** / **Office européen des brevets**

(11) **EP 3 521 425 A1**

(12) **EUROPEAN PATENT APPLICATION**
published in accordance with Art. 153(4) EPC

(43) Date of publication:
**07.08.2019 Bulletin 2019/32**

(51) Int Cl.:
*C12N 15/09* (2006.01)    *C12Q 1/68* (2018.01)
*G06F 19/20* (2011.01)

(21) Application number: **17855657.7**

(22) Date of filing: **07.09.2017**

(86) International application number:
**PCT/JP2017/032287**

(87) International publication number:
**WO 2018/061699 (05.04.2018 Gazette 2018/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2016 JP 2016192158**

(71) Applicant: **FUJIFILM Corporation Tokyo 106-8620 (JP)**

(72) Inventor: **TSUJIMOTO, Takayuki Ashigara-kami-gun Kanagawa 258-8538 (JP)**

(74) Representative: **Klunker IP Patentanwälte PartG mbB Destouchesstraße 68 80796 München (DE)**

(54) **METHOD FOR DESIGNING PRIMERS FOR MULTIPLEX PCR**

(57) There is provided a method for designing primers for multiplex PCR, in which, as a result of designing primers in candidate amplification regions for which priorities are set, if the number of candidate amplification regions in which primers are successfully designed does not reach a desired value, primers can be redesigned while a broad feature of previously set priorities is maintained.

FIG. 2

SEQUENCE LISTING

<110>  Fujifilm Corporation

<120>  Method for redesigning primers for multiplex PCR

<130>  W-6022PCT

<150>  JP2016-192158
<151>  2016-09-29

<160>  2

<170>  PatentIn version 3.5

<210>  1
<211>  20
<212>  DNA
<213>  Artificial Sequence

<220>
<223>  20763642-F

<400>  1
tagccggatg tgggagatgg                                                    20

<210>  2
<211>  20
<212>  DNA
<213>  Artificial Sequence

<220>
<223>  20763642-R

<400>  2
ccagcattgg aaagatctgg                                                    20

**Description**

**BACKGROUND OF THE INVENTION**

1. Field of the Invention

The present invention relates to a method for designing primers for multiplex PCR (Polymerase Chain Reaction).

2. Description of the Related Art

DNA (Deoxyribonucleic acid) sequencers and the like, which have been developed in recent years, facilitate genetic analysis. However, the total base length of the genome is generally enormous, and, on the other hand, sequencers have limited reading capacity. Accordingly, a PCR method is spreading as a technique for efficient and accurate genetic analysis by PCR amplifying only a necessary specific gene region and reading only its base sequence. In particular, a method for selectively PCR amplifying a plurality of gene regions by simultaneously supplying a plurality of types of primers to a certain single PCR reaction system is referred to as multiplex PCR.
Multiplex PCR efficiently PCR amplifies a plurality of regions from a minute amount of DNA and is thus a technique useful for noninvasive prenatal diagnosis.

**SUMMARY OF THE INVENTION**

However, designing a primer set that ensures direct multiplex PCR for minute DNA samples less than or equal to several pg to several tens of pg, such as genomic DNA extracted from a single cell, is of high difficulty due to very restrictive primer design conditions such as complementarity and specificity. Thus, it may be difficult to design primers for PCR amplifying all candidate amplification regions.
The present inventor has shown that when priorities are set for candidate amplification regions and when primers for PCR amplifying the candidate amplification regions are designed according to the priorities, primers for PCR amplifying more candidate amplification regions are likely to be designed than that when priorities are not set for the candidate amplification regions.
However, even when priorities are set for candidate amplification regions, the number of candidate amplification regions in which primers for PCR amplification are successfully designed may be initially smaller than the number of regions necessary for analysis such as genotyping or determination of the number of chromosomes, or, even when the number of candidate amplification regions in which primers for PCR amplification are successfully designed is greater than or equal to the number of regions necessary for analysis, the number of amplification target regions for which PCR amplification products are obtained when multiplex PCR is performed may be smaller than the number of regions necessary for analysis.
In this case, the primers are redesigned. If the previously set priorities are based on a certain intention, it may be desirable to keep the broad feature unchanged.
Accordingly, it is an object of the present invention to provide a method for designing primers for multiplex PCR, in which, as a result of designing primers in candidate amplification regions for which priorities are set, if the number of candidate amplification regions in which primers are successfully designed does not reach a desired value, primers can be redesigned while a broad feature of previously set priorities is maintained.
As a result of intensive studies to solve the problems described above, the present inventor has found that, when primers are to be redesigned, the priorities of the candidate amplification regions are changed to redesign primers. Finally, the present inventor has accomplished the present invention.
That is, the present invention provides the following [1] to [3].

[1] A method for designing primers for multiplex PCR, for amplifying t or more candidate amplification regions among n candidate amplification regions on a genome, including:

a first priority setting step of assigning first priorities from 1 through n to n candidate amplification regions on genomic DNA;
a first primer design step of designing primers for PCR amplifying the candidate amplification regions sequentially in order of the first priorities, starting from a candidate amplification region that is highest of the first priorities;
a first success/failure determination step of determining that designing of primers is complete when $m \geq t$ is satisfied, where $m$ denotes the number of candidate amplification regions in which primers are successfully designed in the first primer design step, and determining that a subsequent step is performed when $m < t$ is satisfied;

a second priority setting step of assigning second priorities from 1 through n to the n candidate amplification regions, the second priorities being in different order than the first priorities; and

a second primer design step of designing primers for PCR amplifying the candidate amplification regions sequentially in order of the second priorities, starting from a candidate amplification region that is highest of the second priorities,

the second priority setting step including the steps of:

inputting identification information and first priority information of the n candidate amplification regions via input means and storing the identification information and the first priority information in storage means;

by arithmetic means, arranging the n candidate amplification regions in order of the first priorities to generate a first sequence including the n candidate amplification regions as elements, and storing the first sequence in the storage means;

by the arithmetic means, segmenting the n candidate amplification regions arranged in order of the first priorities into j blocks so that an i-th block includes $k_i$ candidate amplification regions, and storing the j blocks in the storage means;

by the arithmetic means, rearranging, within at least one block among the j blocks, the candidate amplification regions included in the at least one block, and storing the rearranged candidate amplification regions in the storage means; and

by the arithmetic means, sequentially joining first through j-th blocks together to cancel block segmentation to generate a second sequence, an order of the n candidate amplification regions included in the second sequence being set as an order of the second priorities of the n candidate amplification regions,

where n is an integer satisfying $n \geq 4$, t is an integer satisfying $2 \leq t \leq n$, m is an integer satisfying $0 \leq m \leq n$, i is an integer satisfying $1 \leq i \leq j$, j is an integer satisfying $2 \leq j \leq n/2$, and $k_i$ is an integer satisfying $2 \leq k_i \leq \{n - 2 \times (j - 1)\}$.

[2] The method for designing primers for multiplex PCR according to [1] above, further including, after the second primer design step,

a second success/failure determination step of determining that designing of primers is complete when $m' \geq t$ is satisfied, where m' denotes the number of candidate amplification regions in which primers are successfully designed in the second primer design step, and determining that the second priority setting step is performed again when $m' < t$ is satisfied.

[3] The method for designing primers for multiplex PCR according to claim 1 or 2, wherein in the second priority setting step, an order of the candidate amplification regions within the at least one block is changed randomly.

According to the present invention, it is possible to provide a method for designing primers for multiplex PCR, in which, as a result of designing primers in candidate amplification regions for which priorities are set, if the number of candidate amplification regions in which primers are successfully designed does not reach a desired value, primers can be redesigned while a broad feature of previously set priorities is maintained.

A method for designing primers for multiplex PCR according to the present invention may increase the number of candidate amplification regions in which primers to be used for PCR amplification can be designed, compared with before redesigning is performed, in which case the number of regions necessary for analysis such as genotyping or determination of the number of chromosomes is expected to be ensured.

**BRIEF DESCRIPTION OF THE DRAWINGS**

Fig. 1 is a conceptual diagram illustrating hardware used in a priority setting step according to the present invention;

Fig. 2 is a flow diagram describing an overview of a method for designing primers for multiplex PCR according to the present invention;

Fig. 3 is a diagram illustrating a method for setting second priorities in the method for designing primers for multiplex PCR according to the present invention using a specific example;

Fig. 4 is a flow diagram describing a first aspect of a primer design method after first priorities or second priorities are set in the method for designing primers for multiplex PCR according to the present invention;

Fig. 5 is a flow diagram describing a second aspect of the primer design method after first priorities or second priorities are set in the method for designing primers for multiplex PCR according to the present invention; and

Fig. 6 is a flow diagram describing a third aspect of the primer design method after first priorities or second priorities are set in the method for designing primers for multiplex PCR according to the present invention.

## DESCRIPTION OF THE PREFERRED EMBODIMENTS

In the present invention, a range indicated using "... to ..." refers to a range including values given before and after "to". For example, regarding A and B, "A to B" refers to a range including A and B.

In the present invention, furthermore, a candidate amplification region refers to a candidate region that is a region on a genomic DNA and that is to be PCR amplified for purposes such as genotyping or determination of the number of chromosomes.

In the following, a method for designing primers for multiplex PCR according to the present invention will be described in detail with reference to the drawings, if necessary.

[Hardware (Execution Device)]

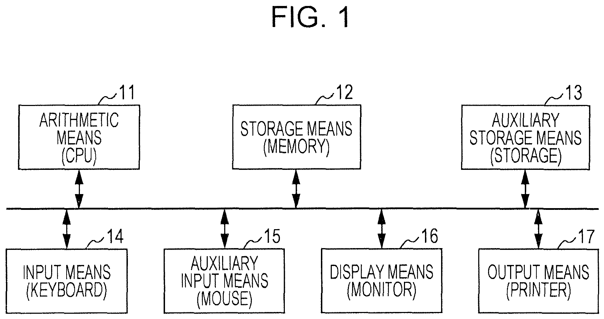

A device (also referred to as "hardware" or "execution device") that executes a priority setting method according to the present invention will be described with reference to Fig. 1.

In the present invention, the setting of priorities is performed by hardware (device) including arithmetic means (CPU; Central Processing Unit) 11, storage means (memory) 12, auxiliary storage means (storage) 13, input means (keyboard) 14, and display means (monitor) 16. This device may further include auxiliary input means (mouse) 15, output means (printer) 17, and so on.

Each means will be described.

The input means (keyboard) 14 is means for inputting instructions, data, and so on to the device. The auxiliary input means (mouse) 15 is used instead of or together with the input means (keyboard) 14.

The arithmetic means (CPU) 11 is means for performing arithmetic processing.

The storage means (memory) 12 is means for storing results of the arithmetic processing performed by the arithmetic means (CPU) 11 or for storing input from the input means (keyboard) 14.

The auxiliary storage means (storage) 13 is a storage that stores an operating system, a program for determining the necessary number of loci, and so on. A portion of the auxiliary storage means (storage) 13 can also be used for extension of the storage means (memory) 12 (virtual memory).

[Method for Designing Primers for Multiplex PCR]

A method for designing primers for multiplex PCR according to the present invention includes the following steps.

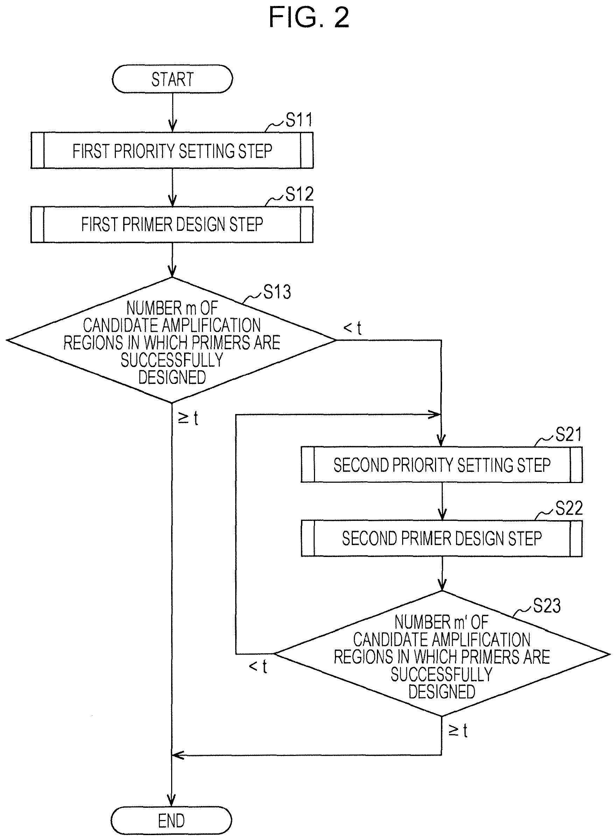

(1) A first priority setting step of assigning first priorities from 1 through n to n candidate amplification regions on genomic DNA ("first priority setting" in Fig. 2), where n is an integer satisfying $n \geq 4$.

(2) A first primer design step of designing primers for PCR amplifying the candidate amplification regions sequentially in order of the first priorities, starting from a candidate amplification region that is highest of the first priorities ("first primer design" in Fig. 2).

(3) A first success/failure determination step of determining that designing of primers is complete when $m \geq t$ is satisfied, where m denotes the number of candidate amplification regions in which primers are successfully designed in the first primer design step, and determining that a subsequent step is performed when $m < t$ is satisfied ("number m of candidate amplification regions in which primers are successfully designed" in Fig. 2), where t is an integer satisfying $2 \leq t \leq n$, and m is an integer satisfying $0 \leq m \leq n$.

(4) A second priority setting step of assigning second priorities from 1 through n to the n candidate amplification regions, the second priorities being in different order than the first priorities ("second priority setting" in Fig. 2).

(5) A second primer design step of designing primers for PCR amplifying the candidate amplification regions sequentially in order of the second priorities, starting from a candidate amplification region that is highest of the second priorities ("second primer design" in Fig. 2).

In addition, the following step may also be included, if desired.

(6) A second success/failure determination step of determining that designing of primers is complete when $m' \geq t$ is satisfied, where m' denotes the number of candidate amplification regions in which primers are successfully designed in the second primer design step, and determining that the second priority setting step is performed again when $m' < t$ is satisfied ("number m' of candidate amplification regions in which primers are successfully designed" in Fig. 2).

In the following, each step will be described.

<First Priority Setting Step S11>

In Fig. 2, this step is represented as "first priority setting step".

In first priority setting step S11, n candidate amplification regions are assigned numbers from 1 to n without overlap. The order of the numbers is an order in which primers are designed.

The way to assign numbers is not specifically limited, but is as follows, for example.

«Specific Example (1) of First Priority Setting Method»

Identification information and coordinate information of n candidate amplification regions on the same chromosomal DNA are input via the input means and are stored in the storage means 12.

The arithmetic means 11 searches for a candidate amplification region having a minimum coordinate value by using the identification information and coordinate information of the candidate amplification regions stored in the storage means, assigns priority information indicating a priority of 1, which corresponds to the highest priority, to the found candidate amplification region, and stores the priority information in the storage means 12.

The arithmetic means 11 searches for a candidate amplification region having a maximum coordinate value by using the identification information and coordinate information of the candidate amplification regions stored in the storage means, assigns priority information indicating a priority of 2, which corresponds to the second highest priority, to the found candidate amplification region, and stores the priority information in the storage means 12.

The arithmetic means 11 searches for a candidate amplification region $R_i$ and a candidate amplification region Rj by using the identification information, coordinate information, and priority information of the candidate amplification regions stored in the storage means, the candidate amplification region $R_i$ and the candidate amplification region $R_j$ being respectively a candidate amplification region whose priority is i, whose coordinate value is $r_i$, and whose identification name is $R_i$ and a candidate amplification region whose priority is j, whose coordinate value is $r_j$, and whose identification name is $R_j$ and satisfying a condition that no candidate amplification region assigned a priority is present but at least one candidate amplification region yet to be assigned a priority is present between the candidate amplification region $R_i$ and the candidate amplification region $R_j$, then calculates a coordinate value $r_{i-j}$ of a midpoint of the candidate amplification region $R_i$ and the candidate amplification region Rj in accordance with $r_{i-j} = (r_i + r_j)/2$, further searches for a candidate amplification region having a coordinate value closest to the coordinate value $r_{i-j}$ of the midpoint, assigns priority information indicating a priority of k, which corresponds to the k-th highest priority, to the found candidate amplification region, and stores the priority information in the storage means 12.

The step of assigning a priority of k is repeated for k = 3 to n.

Accordingly, first priorities can be set.

Priorities may be set in the following way.

The arithmetic means 11 searches for a candidate amplification region having a maximum coordinate value by using the identification information and coordinate information of the candidate amplification regions stored in the storage means, assigns priority information indicating a priority of 1, which corresponds to the highest priority, to the found candidate amplification region, and stores the priority information in the storage means 12.

The arithmetic means 11 searches for a candidate amplification region having a minimum coordinate value by using the identification information and coordinate information of the candidate amplification regions stored in the storage means, assigns priority information indicating a priority of 2, which corresponds to the second highest priority, to the found candidate amplification region, and stores the priority information in the storage means 12.

Note that n is an integer satisfying $3 \leq n$, k is an integer satisfying $3 \leq k \leq n$, i and j satisfy $1 \leq i \leq k - 1$, $1 \leq j \leq k - 1$, and $i \neq j$, $r_i$ and $r_j$ satisfy $rmin \leq r_i \leq r_{max}$, $rmin \leq r_j \leq r_{max}$, and $r_i \neq r_j$, and rmin and $r_{max}$ are respectively a minimum coordinate value and a maximum coordinate value of the n candidate amplification regions.

The specific example (1) of the priority setting method described above may be described as follows.

In the n candidate amplification regions, a candidate amplification region having the minimum coordinate value $r_{min}$ is represented by $R_{min}$, and a candidate amplification region having the maximum coordinate value $r_{max}$ is represented by $R_{max}$.

First, one of the two candidate amplification regions, namely, the candidate amplification region $R_{min}$ and the candidate amplification region $R_{max}$, is assigned a priority of 1, which corresponds to the highest priority. That is, the candidate amplification region $R_{min}$ is assigned a priority of 1, or the candidate amplification region $R_{max}$ is assigned a priority of 1.

Then, the other of the two candidate amplification regions, namely, the candidate amplification region $R_{min}$ and the candidate amplification region $R_{max}$, except for the one assigned a priority of 1, is assigned a priority of 2, which corresponds to the second highest priority. That is, when the candidate amplification region $R_{min}$ is assigned a priority of 1, the candidate amplification region $R_{max}$ is assigned a priority of 2. When the candidate amplification region $R_{max}$ is assigned a priority of 1, the candidate amplification region $R_{min}$ is assigned a priority of 2.

Further, the third through h-th candidate amplification regions are assumed to have already been assigned priorities from 1 through (h - 1), and a candidate amplification region having the coordinate value closest to a coordinate value $(r_p + r_q)/2$ of a midpoint of a candidate amplification region $R_p$ assigned a priority of p and a candidate amplification region $R_q$ assigned a priority of q is assigned a priority of h. Here, $r_p$ and $r_q$ are coordinate values of the candidate amplification region $R_p$ and the candidate amplification region $R_q$, respectively.

When two or more combinations of the candidate amplification region $R_p$ and the candidate amplification region $R_q$ are present, one combination may be randomly selected. Alternatively, a policy may be employed such that, for example, one of them having a smaller coordinate value is given precedence or one of them having a larger coordinate value is given precedence.

When two candidate amplification regions having the coordinate value closest to the coordinate value $(r_p + r_q)/2$ are present, one region may be randomly selected. Alternatively, a policy may be employed such that, for example, one of them having a smaller coordinate value is given precedence or one of them having a larger coordinate value is given precedence.

Note that no candidate amplification region assigned a priority is present but at least one candidate amplification region yet to be assigned a priority is present between the candidate amplification region $R_p$ and the candidate amplification region $R_q$. Further, $R_i$ is $R_{min}$ or $R_{max}$ assigned a priority of 1, and $R_2$ is $R_{min}$ or $R_{max}$ assigned a priority of 2.

h is an integer satisfying $3 \leq h \leq n$.

p and q satisfy $1 \leq p \leq h-1$, $1 \leq q \leq h-1$, and $p \neq q$.

$r_p$ and $r_q$ satisfy $r_{min} \leq r_p \leq r_{max}$, $r_{min} \leq r_q \leq r_{max}$, and $r_p \neq r_q$.

An h-th priority setting step is repeated sequentially for h = 3 to h = n.

When there is no candidate amplification region that can be assigned a priority, the setting of priorities is complete.

Note that priorities are set for the candidate amplification regions so that the priorities do not overlap.


«Specific Example (2) of First priority setting method»


Identification information and coordinate information of n candidate amplification regions on the same chromosomal DNA are input via the input means and are stored in the storage means 12.

The arithmetic means 11 searches for a candidate amplification region having a minimum coordinate value by using the identification information and coordinate information of the candidate amplification regions stored in the storage means, assigns priority information indicating a priority of 1, which corresponds to the highest priority, to the found candidate amplification region, and stores the priority information in the storage means 12.

The arithmetic means 11 searches for an identification name $R_{k-1}$ and a coordinate value $r_{k-1}$ of a candidate amplification region whose priority is k - 1 by using the identification information, coordinate information, and priority information of the candidate amplification regions stored in the storage means, calculates $T = r_{k-1} + t$, when $T = r_{k-1} + t \leq r_{max}$ is satisfied, searches for a candidate amplification region yet to be assigned priority information and having a coordinate value greater than or equal to $r_{k-1} + t$ and less than or equal to $r_{max}$, if a candidate amplification region satisfying these conditions is present, assigns a priority of k to a candidate amplification region yet to be assigned priority information and having the smallest coordinate value greater than or equal to $r_{k-1} + t$, and stores the candidate amplification region in the storage means 12, when $T = r_{k-1} + t \leq r_{max}$ is satisfied, searches for a candidate amplification region yet to be assigned priority information and having a coordinate value greater than or equal to $r_{k-1} + t$ and less than or equal to $r_{max}$, if there is no candidate amplification region satisfying these conditions, assigns a priority of k to a candidate amplification region yet to be assigned priority information and having the smallest coordinate value greater than or equal to rmin, and stores the candidate amplification region in the storage means 12, and when $T = r_{k-1} + t > r_{max}$ is satisfied, assigns a priority of k to a candidate amplification region yet to be assigned a priority and having the smallest coordinate value greater than or equal to rmin, and stores the candidate amplification region in the storage means 12.

The step of assigning a priority of k is repeated for k = 2 to n.

Accordingly, first priorities can be set.

Priorities may be set in the following way.

The arithmetic means 11 searches for a candidate amplification region having a maximum coordinate value by using the identification information and coordinate information of the candidate amplification regions stored in the storage means, assigns priority information indicating a priority of 1, which corresponds to the highest priority, to the found candidate amplification region, and stores the priority information in the storage means 12.

Note that n is an integer satisfying $3 \leq n$, k is an integer satisfying $2 \leq k \leq n$, t is a real number satisfying t > 0, $r_{k-1}$ # rk is satisfied, and $r_{min}$ and $r_{max}$ are respectively a minimum coordinate value and a maximum coordinate value of the n candidate amplification regions.

The specific example (2) of the first priority setting method may be described as follows.

In the n candidate amplification regions, a candidate amplification region having the minimum coordinate value rmin is represented by $R_{min}$, and a candidate amplification region having the maximum coordinate value $r_{max}$ is represented by $R_{max}$.

First, a candidate amplification region $R_1$ located between the two candidate amplification regions described above, namely, the candidate amplification region $R_{min}$ and the candidate amplification region $R_{max}$, and having a coordinate value $r_1$ satisfying $rmin \leq r_1 \leq r_{max}$ is assigned a priority of 1, which corresponds to the highest priority. That is, the candidate amplification region $R_{min}$ may be assigned a priority of 1, the candidate amplification region $R_{max}$ may be assigned a priority of 1, or a candidate amplification region different from the candidate amplification region $R_{min}$ and the candidate amplification region $R_{max}$ may be assigned a priority of 1.

The coordinate value $r_1$ of $R_1$ is not specifically limited so long as it satisfies $r_{min} \leq r_1 \leq r_{max}$, but preferably satisfies $r_1 = rmin$ or $r_1 = r_{max}$, and more preferably satisfies $r_1 = r_{min}$.

Further, priorities from 1 through (h - 1) are assumed to have already been set, and a candidate amplification region satisfying predetermined conditions is assigned a priority of h.

A candidate amplification region satisfying the predetermined conditions is determined in the following way.

Consideration is given to value "$S = r_{h-1} + s$", which is obtained by adding a threshold value s to $r_{h-1}$, where $r_{h-1}$ denotes the coordinate value of a candidate amplification region $R_{h-1}$ assigned a priority of (h - 1). Here, s is a real number satisfying $s > 0$, which is referred to sometimes as "threshold value" in the present invention.

Since the maximum coordinate value of the candidate amplification regions is represented by $r_{max}$, the following two cases (1) and (2) are obtained.

$$(1) \text{ A case where } S = r_{h-1} + s \leq r_{max} \text{ is satisfied.}$$

$$(2) \text{ A case where } S = r_{h-1} + s > r_{max} \text{ is satisfied.}$$

The case (1) is further divided into the following two cases (1)-1 and (1)-2 in accordance with whether a candidate amplification region yet to be assigned a priority is present between the coordinate value $r_{h-1} + s$ and $r_{max}$.

(1)-1 A case where a candidate amplification region yet to be assigned a priority is present.
(1)-2 A case where a candidate amplification region yet to be assigned a priority is not present.

When (1)-1 is satisfied, a candidate amplification region yet to be assigned a priority and having the smallest coordinate value greater than or equal to ($r_{h-1} + s$) is assigned a priority of h.

In this case, s denotes the distance between the candidate amplification region $R_{h-1}$ having a priority of (h - 1) and a candidate amplification region $R_h$ having a priority of h. As s increases, the distance between $R_{h-1}$ and $R_h$ also increases, generally reducing the effect of $R_{h-1}$ and $R_h$ on each other.

When (1)-2 or (2) is satisfied, a candidate amplification region yet to be assigned a priority and having the smallest coordinate value greater than or equal to $r_{min}$ is assigned a priority of h.

h is an integer satisfying $2 \leq h \leq n$.

s is a real number satisfying $s > 0$, which can be set as appropriate in accordance with the chromosomal DNA size, the coordinates of the candidate amplification regions, or the like, and is preferably 100,000 or more, more preferably 1,000,000 or more, and even more preferably 5,000,000 or more.

The h-th priority setting step is repeated sequentially for h = 2 to h = n.

When there is no candidate amplification region that can be assigned a priority, the setting of priorities is complete.

Note that priorities are set for the candidate amplification regions so that the priorities do not overlap.

<First Primer Design Step S12/Second Primer Design Step S22>

In Fig. 2, these steps are represented as "first primer design step" and "second primer design step".

A description will be provided in "primer design method after first priorities or second priorities are set" described below.

In the first primer design step, first priorities may be set midway. For example, in a third aspect provided in "primer design method after first priorities or second priorities are set" described below, after base sequences of candidate primers are designed in all n candidate amplification regions, first priorities may be set, and primers may be selected sequentially from the candidate primers in order of the first priorities.

<First Success/Failure Determination Step S13/Second Success/Failure Determination Step S23>

In Fig. 2, these steps are represented as "number m of candidate amplification regions in which primers are successfully designed" and "number m' of candidate amplification regions in which primers are successfully designed".

In first success/failure determination step S13, when $m \geq t$ is satisfied, where m denotes the number of candidate amplification regions in which primers are successfully designed in first primer design step S12, it is determined that designing of primers is complete, and when $m < t$ is satisfied, it is determined that a subsequent step is performed.

In second success/failure determination step S23, when $m' \geq t$ is satisfied, where m' denotes the number of candidate amplification regions in which primers are successfully designed in second primer design step S22, it is determined that designing of primers is complete, and when $m' < t$ is satisfied, it is determined that a subsequent step is performed.

t is an integer satisfying $2 \leq t \leq n$. The value t is a target value of candidate amplification regions in which primers are successfully designed, and can be set as appropriate in accordance with the purpose of analysis such as genotyping or determination of the number of chromosomes.

m is an integer satisfying $0 \leq m \leq n$, and m' is an integer satisfying $0 \leq m' \leq n$. The values m and m' are actual values of candidate amplification regions in which primers are successfully designed in the first primer design step S12 or the second primer design step S22. In the present invention, when $m < t$ is satisfied, the primers are redesigned so that $m' \geq t$ is satisfied.

<Second Priority Setting Step S21>

In Fig. 2, this step is represented as "second priority setting step".

Second priority setting step S21 is a step including a step of inputting identification information and first priority information of n candidate amplification regions via the input means 14 and storing the identification information and the first priority information in the storage means 12, a step of, by the arithmetic means 11, extracting n candidate amplification regions from the group consisting of the n candidate amplification regions, arranging the n candidate amplification regions in order of the first priorities to generate a first sequence including the n candidate amplification regions as elements, and storing the first sequence in the storage means 12, a step of, by the arithmetic means 11, segmenting the n candidate amplification regions arranged in order of the first priorities into j blocks so that the i-th block includes $k_i$ candidate amplification regions, and storing the j blocks in the storage means 12, a step of, by the arithmetic means 11, within at least one block among the j blocks, rearranging candidate amplification regions included in the at least one block, and storing the rearranged candidate amplification regions in the storage means 12, a step of, by the arithmetic means 11, sequentially joining the first through j-th blocks together to cancel block segmentation, and storing the joined block in the storage means 12, and a step of, by the arithmetic means 11, generating a second sequence including the n candidate amplification regions as elements and storing the second sequence in the storage means 12, in which the order of the n candidate amplification regions included in the second sequence is set as the order of the second priorities of the n candidate amplification regions.

Note that n is an integer satisfying $n \geq 4$, t is an integer satisfying $2 \leq t \leq n$, m is an integer satisfying $0 \leq m \leq n$, i is an integer satisfying $1 \leq i \leq j$, j is an integer satisfying $2 \leq j \leq n/2$, and $k_i$ is an integer satisfying $2 \leq k_i \leq \{n - 2 \times (j - 1)\}$.

In the second priority setting step S21, the method of changing the order of the candidate amplification regions within the at least one block is not specifically limited, but the order of the candidate amplification regions is preferably changed randomly by using random shuffling, random permutation, or the like. Further, since the number of candidate amplification regions included in a single block is finite, an algorithm for generating a random sequence from finite elements can be utilized. Examples of the algorithm include the Fisher-Yates shuffle.

Further, the number of blocks into which candidate amplification regions are segmented is not limited to any specific value so long as it is two or more, and is preferably set to about 10.

Further, the number of candidate amplification regions included in a single block is not limited to any specific value so long as it is two or more, and is preferably about 1/10 of the total number of candidate amplification regions. Further, the difference between the numbers of candidate amplification regions included in blocks preferably falls within 1 to 5, and more preferably falls within 1 to 3.

«Description based on Specific Example of Second Priority Setting Step»

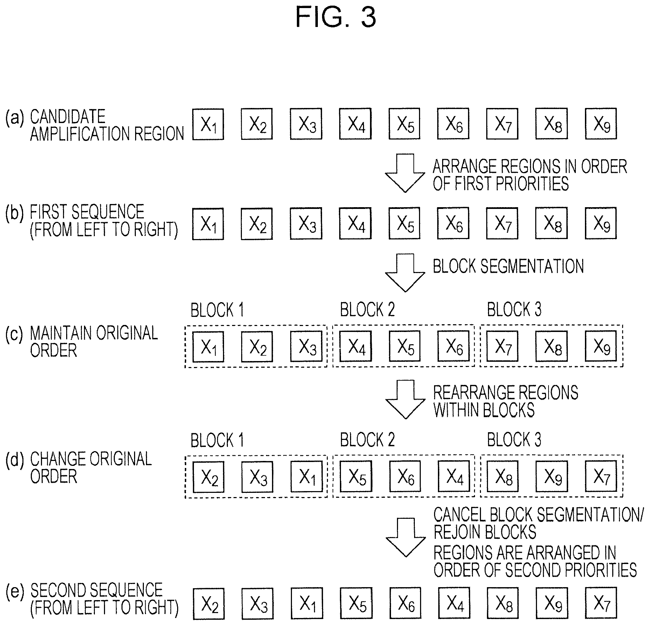

In the following, the second priority setting step S21 will be described in more detail with reference to Fig. 3. Note that this specific example is not limiting.

Part (a) of Fig. 3 illustrates nine candidate amplification regions $X_1$ to $X_9$.

First, as illustrated in part (b) of Fig. 3, the nine candidate amplification regions $X_1$ to $X_9$ are arranged according to the first priorities. Since priorities are assigned in order from lowest to highest in terms of coordinate value, the candidate amplification regions $X_1$ to $X_9$ are arranged in this order.

Then, as illustrated in part (c) of Fig. 3, the nine candidate amplification regions $X_1$ to $X_9$ are segmented into three blocks in such a manner that each block includes three candidate amplification regions. Here, the nine candidate

amplification regions $X_1$ to $X_9$ are segmented in such a manner that the first block includes the three candidate amplification regions $X_1$ to $X_3$, the second block includes the three candidate amplification regions $X_4$ to $X_6$, and the third block includes the three candidate amplification regions $X_7$ to $X_9$.

Then, as illustrated in part (d) of Fig. 3, the order of the candidate amplification regions in each block is changed.

Then, as illustrated in part (e) of Fig. 3, block segmentation is canceled without changing the order of the blocks to obtain a new sequence of the candidate amplification regions. The order of the numbers identified in this sequence is the order of second priorities.

[Primer Design Method after First Priorities or Second Priorities Are Set]

In the method for designing primers for multiplex PCR according to the present invention, a primer design method after the first priorities or the second priorities are set (hereinafter, simply, "primer design method after priority setting") is not specifically limited, and is preferably selected from three aspects described below. In this case, the primer design method after the first priorities are set and the primer design method after the second priorities are set may be performed in the same way or in different ways.

<First Aspect of Primer Design Method after Priority Setting>

A first aspect of the primer design method after priority setting (referred to sometimes as "first aspect") includes the following: (a) a target region selection step, (b) a candidate primer base sequence generation step, (c) a local alignment step, (d) a first-stage selection step, (e) a global alignment step, (f) a second-stage selection step, and (g) a primer employment step.

(a) A target region selection step of selecting a target region from candidate amplification regions with set priorities in order of priority.

(b) A candidate primer base sequence generation step of generating at least one base sequence of a candidate primer for PCR amplifying the target region on the basis of each of base sequences of respective neighboring regions located at two ends of the target region on genomic DNA.

(c) A local alignment step of performing pairwise local alignment, for all combinations for selecting base sequences of two candidate primers from among base sequences of candidate primers generated in the candidate primer base sequence generation step, on two base sequences included in each of the combinations, under a condition in which partial sequences to be compared for the two base sequences include 3'-ends of the two base sequences, to determine local alignment scores.

(d) A first-stage selection step of performing first-stage selection of base sequences of candidate primers for PCR amplifying the target region on the basis of the local alignment scores.

(e) A global alignment step of performing pairwise global alignment, for all combinations for selecting base sequences of two candidate primers from among base sequences of candidate primers selected in the first-stage selection step, on base sequences having a preset sequence length and including 3'-ends of two base sequences included in each of the combinations, to determine global alignment scores.

(f) A second-stage selection step of performing second-stage selection of base sequences of candidate primers for PCR amplifying the target region on the basis of the global alignment scores.

(g) A primer employment step of employing, as base sequences of primers for PCR amplifying the target region, base sequences of candidate primers selected in both the first-stage selection step and the second-stage selection step.

Among the steps (a) to (g), both the steps (c) and (d) and both the steps (e) and (f) may be performed in any order or performed simultaneously. That is, the steps (e) and (f) may be performed after the steps (c) and (d) are performed, or the steps (c) and (d) may be performed after the steps (e) and (f) are performed. Alternatively, the steps (c) and (d) and the steps (e) and (f) may be performed in parallel.

If the steps (c) and (d) are performed after the steps (e) and (f) are performed, the steps (e) and (c) are preferably replaced with steps (e') and (c') below, respectively.

(e') A global alignment step of performing pairwise global alignment, for all combinations for selecting base sequences of two candidate primers from among base sequences of candidate primers generated in the candidate primer base sequence generation step, on base sequences having a preset sequence length and including 3'-ends of two base sequences included in each of the combinations, to determine global alignment scores.

(c') A local alignment step of performing pairwise local alignment, for all combinations for selecting base sequences of two candidate primers from among base sequences of candidate primers selected in the second-stage selection step, on two base sequences included in each of the combinations, under a condition in which partial sequences to be compared for the two base sequences include 3'-ends of the two base sequences, to determine local alignment scores.

Further, if the steps (c) and (d) and the steps (e) and (f) are performed in parallel, the step (e) is preferably replaced with step (e') below.

(e') A global alignment step of performing pairwise global alignment, for all combinations for selecting base sequences of two candidate primers from among base sequences of candidate primers generated in the candidate primer base sequence generation step, on base sequences having a preset sequence length and including 3'-ends of two base sequences included in each of the combinations, to determine global alignment scores.

<Second Aspect of Primer Design Method after Priority Setting>

A second aspect of the primer design method after priority setting (referred to sometimes as "second aspect") includes the following: ($a_1$) a first step of target region selection, ($b_1$) a first step of candidate primer base sequence generation, ($c_1$) a first step of local alignment, ($d_1$) a first step of first-stage selection, ($e_1$) a first step of global alignment, ($f_1$) a first step of second-stage selection, ($g_1$) a first step of primer employment, ($a_2$) a second step of target region selection, ($b_2$) a second step of candidate primer base sequence generation, ($c_2$) a second step of local alignment, ($d_2$) a second step of first-stage selection, ($e_2$) a second step of global alignment, ($f_2$) a second step of second-stage selection, and ($g_2$) a second step of primer employment.

($a_1$) A first step of target region selection for selecting the candidate amplification region having the highest priority as a first target region from among the candidate amplification regions with set priorities.

($b_1$) A first step of candidate primer base sequence generation for generating at least one base sequence of a candidate primer for PCR amplifying the first target region on the basis of each of base sequences of respective neighboring regions located at two ends of the first target region on genomic DNA.

($c_1$) A first step of local alignment for performing pairwise local alignment, for all combinations for selecting base sequences of two candidate primers from among base sequences of candidate primers generated in the first step of candidate primer base sequence generation, on two base sequences included in each of the combinations, under a condition in which partial sequences to be compared for the two base sequences include 3'-ends of the two base sequences, to determine local alignment scores.

($d_1$) A first step of first-stage selection for performing first-stage selection of base sequences of candidate primers for PCR amplifying the first target region on the basis of the local alignment scores.

($e_1$) A first step of global alignment for performing pairwise global alignment, for all combinations for selecting base sequences of two candidate primers from among base sequences of candidate primers selected in the first step of first-stage selection, on base sequences having a preset sequence length and including 3'-ends of two base sequences included in each of the combinations, to determine global alignment scores.

($f_1$) A first step of second-stage selection for performing second-stage selection of base sequences of candidate primers for PCR amplifying the first target region on the basis of the global alignment scores.

($g_1$) A first step of primer employment for employing, as base sequences of primers for PCR amplifying the first target region, base sequences of candidate primers selected in both the first step of first-stage selection and the first step of second-stage selection.

($a_2$) A second step of target region selection for selecting, as a second target region, a candidate amplification region having the highest priority from among candidate amplification regions that have not been selected among candidate amplification regions with set priorities.

($b_2$) A second step of candidate primer base sequence generation for generating at least one base sequence of a candidate primer for PCR amplifying the second target region on the basis of each of base sequences of respective neighboring regions located at two ends of the second target region on genomic DNA.

($c_2$) A second step of local alignment for performing pairwise local alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on two base sequences included in each of the combinations, under a condition in which partial sequences to be compared for the two base sequences include 3'-ends of the two base sequences, to determine local alignment scores.

(d2) A second step of first-stage selection for performing first-stage selection of base sequences of candidate primers for PCR amplifying the second target region on the basis of the local alignment scores.

($e_2$) A second step of global alignment for performing pairwise global alignment, for all combinations for selecting base sequences of two candidate primers and all combinations for selecting a base sequence of one candidate primer and a base sequence of one primer that has already been employed from among base sequences of candidate primers selected in the second step of first-stage selection and from among base sequences of primers that have already been employed, on base sequences having a preset sequence length and including 3'-ends of two base sequences included in each of the combinations, to determine global alignment scores.

($f_2$) A second step of second-stage selection for performing second-stage selection of base sequences of candidate primers for PCR amplifying the second target region on the basis of the global alignment scores.

($g_2$) A second step of primer employment for employing, as base sequences of primers for PCR amplifying the second target region, base sequences of candidate primers selected in both the second step of first-stage selection and the second step of second-stage selection.

Among the steps ($a_1$) to ($g_1$), both the steps ($c_1$) and ($d_1$) and both the steps ($e_1$) and ($f_1$) may be performed in any order or performed simultaneously. That is, the steps ($e_1$) and ($f_1$) may be performed after the steps ($c_1$) and ($d_1$) are performed, or the steps ($c_1$) and ($d_1$) may be performed after the steps ($e_1$) and ($f_1$) are performed. Alternatively, the steps ($c_1$) and ($d_1$) and the steps ($e_1$) and ($f_1$) may be performed in parallel.

If the steps ($c_1$) and ($d_1$) are performed after the steps ($e_1$) and ($f_1$) are performed, the steps ($e_1$) and ($c_1$) are preferably replaced with steps ($e_1$') and ($c_1$') below, respectively.

($e_1$') A first step of global alignment for performing pairwise global alignment, for all combinations for selecting base sequences of two candidate primers from among base sequences of candidate primers generated in the first step of candidate primer base sequence generation, on base sequences having a preset sequence length and including 3'-ends of two base sequences included in each of the combinations, to determine global alignment scores.

($c_1$') A first step of local alignment for performing pairwise local alignment, for all combinations for selecting base sequences of two candidate primers from among base sequences of candidate primers selected in the first step of second-stage selection, on two base sequences included in each of the combinations, under a condition in which partial sequences to be compared for the two base sequences include 3'-ends of the two base sequences, to determine local alignment scores.

Further, if the steps ($c_1$) and ($d_1$) and the steps ($e_1$) and ($f_1$) are performed in parallel, the step ($e_1$) is preferably replaced with step ($e_1$') below.

($e_1$') A first step of global alignment for performing pairwise global alignment, for all combinations for selecting base sequences of two candidate primers from among base sequences of candidate primers generated in the first step of candidate primer base sequence generation, on base sequences having a preset sequence length and including 3'-ends of two base sequences included in each of the combinations, to determine global alignment scores.

Among the steps ($a_2$) to ($g_2$), both the steps ($c_2$) and ($d_2$) and both the steps ($e_2$) and ($f_2$) may be performed in any order or performed simultaneously. That is, the steps ($e_2$) and ($f_2$) may be performed after the steps ($c_2$) and ($d_2$) are performed, or the steps ($c_2$) and ($d_2$) may be performed after the steps ($e_2$) and ($f_2$) are performed. Alternatively, the steps ($c_2$) and ($d_2$) and the steps ($e_2$) and ($f_2$) may be performed in parallel.

If the steps ($c_2$) and ($d_2$) are performed after the steps ($e_2$) and ($f_2$) are performed, the steps ($e_2$) and ($c_2$) are preferably replaced with steps ($e_2$') and ($c_2$') below, respectively.

($e_2$') A second step of global alignment for performing pairwise global alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on base sequences having a preset sequence length and including 3'-ends of two base sequences included in each of the combinations, to determine global alignment scores.

($c_2$') A second step of local alignment for performing pairwise local alignment, for all combinations for selecting base sequences of two candidate primers and all combinations for selecting a base sequence of one candidate primer and a base sequence of one primer that has already been employed from among base sequences of candidate primers selected in the second step of second-stage selection and from among base sequences of primers that have already been employed, on two base sequences included in each of the combinations, under a condition in which partial sequences to be compared for the two base sequences include 3'-ends of the two base sequences, to determine local alignment scores.

Further, if the steps ($c_2$) and ($d_2$) and the steps ($e_2$) and ($f_2$) are performed in parallel, the step ($e_2$) is preferably replaced with step ($e_2$') below.

($e_2$') A second step of global alignment for performing pairwise global alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on base sequences having a preset sequence length and including 3'-ends of two base sequences included in each of the combinations, to determine global alignment scores.

Further, when the at least one region of interest includes three or more regions of interest and when base sequences of primers for PCR amplifying third and subsequent target regions that have not yet been selected from the three or more regions of interest are employed, the steps $(a_2)$ to $(g_2)$ are repeated for each of the third and subsequent target regions.

<Third Aspect of Primer Design Method after Priority Setting>

A third aspect of the primer design method after priority setting (referred to sometimes as "third aspect") includes the following: (a-0) a plurality-of-target-region selection step, (b-0) a plurality-of-candidate-primer-base-sequence generation step, (c-1) a first local alignment step, (d-1) a first first-stage selection step, (e-1) a first global alignment step, (f-1) a first second-stage selection step, (g-1) a first primer employment step, (c-2) a second local alignment step, (d-2) a second first-stage selection step, (e-2) a second global alignment step, (f-2) a second second-stage selection step, and (g-2) a second primer employment step.

(a-0) A plurality-of-target-region selection step of selecting a plurality of target regions from candidate amplification regions with set priorities in order from highest to lowest in terms of priority.

(b-0) A plurality-of-candidate-primer-base-sequence generation step of generating at least one base sequence of a candidate primer for PCR amplifying each of the plurality of target regions on the basis of each of base sequences of respective neighboring regions located at two ends of each of the plurality of target regions on genomic DNA.

(c-1) A first local alignment step of performing pairwise local alignment, for all combinations for selecting base sequences of two candidate primers from among base sequences of candidate primers for PCR amplifying a first target region having the highest priority among base sequences of candidate primers generated in the plurality-of-candidate-primer-base-sequence generation step, on two base sequences included in each of the combinations, under a condition in which partial sequences to be compared for the two base sequences include 3'-ends of the two base sequences, to determine local alignment scores.

(d-1) A first first-stage selection step of performing first-stage selection of base sequences of candidate primers for PCR amplifying the first target region on the basis of the local alignment scores.

(e-1) A first global alignment step of performing pairwise global alignment, for all combinations for selecting base sequences of two candidate primers from among base sequences of candidate primers selected in the first first-stage selection step, on base sequences having a preset sequence length and including 3'-ends of two base sequences included in each of the combinations, to determine global alignment scores.

(f-1) A first second-stage selection step of performing second-stage selection of base sequences of candidate primers for PCR amplifying the first target region on the basis of the global alignment scores.

(g-1) A first primer employment step of employing, as base sequences of primers for PCR amplifying the first target region, base sequences of candidate primers selected in both the first first-stage selection step and the first second-stage selection step.

(c-2) A second local alignment step of performing pairwise local alignment, for all combinations for selecting base sequences of two candidate primers and all combinations for selecting a base sequence of one candidate primer and a base sequence of one primer that has already been employed from among base sequences of candidate primers for PCR amplifying a second target region having a priority of 2 among base sequences of candidate primers generated in the plurality-of-candidate-primer-base-sequence generation step and from among base sequences of primers that have already been employed, on two base sequences included in each of the combinations, under a condition in which partial sequences to be compared for the two base sequences include 3'-ends of the two base sequences, to determine local alignment scores.

(d-2) A second first-stage selection step of performing first-stage selection of base sequences of candidate primers for PCR amplifying the second target region on the basis of the local alignment scores.

(e-2) A second global alignment step of performing pairwise global alignment, for all combinations for selecting base sequences of two candidate primers and all combinations for selecting a base sequence of one candidate primer and a base sequence of one primer that has already been employed from among base sequences of candidate primers selected in the second first-stage selection step and from among base sequences of primers that have already been employed, on base sequences having a preset sequence length and including 3'-ends of two base sequences included in each of the combinations, to determine global alignment scores.

(f-2) A second second-stage selection step of performing second-stage selection of base sequences of candidate primers for PCR amplifying the second target region on the basis of the global alignment scores.
(g-2) A second primer employment step of employing, as base sequences of primers for PCR amplifying the second target region, base sequences of candidate primers selected in both the second first-stage selection step and the second second-stage selection step.

Among the steps (c-1) to (g-1), both the steps (c-1) and (d-1) and both the steps (e-1) and (f-1) may be performed in any order or performed simultaneously. That is, the steps (e-1) and (f-1) may be performed after the steps (c-1) and (d-1) are performed, or the steps (c-1) and (d-1) may be performed after the steps (e-1) and (f-1) are performed. Alternatively, the steps (c-1) and (d-1) and the steps (e-1) and (f-1) may be performed in parallel.
If the steps (c-1) and (d-1) are performed after the steps (e-1) and (f-1) are performed, the steps (e-1) and (c-1) are preferably replaced with steps (e'-1) and (c'-1) below, respectively.

(e'-1) A first global alignment step of performing pairwise global alignment, for all combinations for selecting base sequences of two candidate primers from among base sequences of candidate primers for PCR amplifying a first target region having the highest priority among base sequences of candidate primers generated in the plurality-of-candidate-primer-base-sequence generation step, on base sequences having a preset sequence length and including 3'-ends of two base sequences included in each of the combinations, to determine global alignment scores.
(c'-1) A first local alignment step of performing pairwise local alignment, for all combinations for selecting base sequences of two candidate primers from among base sequences of candidate primers selected in the first second-stage selection step, on two base sequences included in each of the combinations, under a condition in which partial sequences to be compared for the two base sequences include 3'-ends of the two base sequences, to determine local alignment scores.

Further, if the steps (c-1) and (d-1) and the steps (e-1) and (f-1) are performed in parallel, the step (e-1) is preferably replaced with step (e'-1) below.
(e'-1) A first global alignment step of performing pairwise global alignment, for all combinations for selecting base sequences of two candidate primers from among base sequences of candidate primers for PCR amplifying a first target region having the highest priority among base sequences of candidate primers generated in the plurality-of-candidate-primer-base-sequence generation step, on base sequences having a preset sequence length and including 3'-ends of two base sequences included in each of the combinations, to determine global alignment scores.
Among the steps (c-2) to (g-2), both the steps (c-2) and (d-2) and both the steps (e-2) and (f-2) may be performed in any order or performed simultaneously. That is, the steps (e-2) and (f-2) may be performed after the steps (c-2) and (d-2) are performed, or the steps (c-2) and (d-2) may be performed after the steps (e-2) and (f-2) are performed. Alternatively, the steps (c-1) and (d-1) and the steps (e-1) and (f-1) may be performed in parallel.
If the steps (c-2) and (d-2) are performed after the steps (e-2) and (f-2) are performed, the steps (e-2) and (c-2) are preferably replaced with steps (e'-2) and (c'-2) below, respectively. (e'-2) A second global alignment step of performing pairwise global alignment, for all combinations for selecting base sequences of two candidate primers and all combinations for selecting a base sequence of one candidate primer and a base sequence of one primer that has already been employed from among base sequences of candidate primers for PCR amplifying a second target region having a priority of 2 among base sequences of candidate primers generated in the plurality-of-candidate-primer-base-sequence generation step and from among base sequences of primers that have already been employed, on base sequences having a preset sequence length and including 3'-ends of two base sequences included in each of the combinations, to determine global alignment scores.
(c'-2) A second local alignment step of performing pairwise local alignment, for all combinations for selecting base sequences of two candidate primers and all combinations for selecting a base sequence of one candidate primer and a base sequence of one primer that has already been employed from among base sequences of candidate primers selected in the second second-stage selection step and from among base sequences of primers that have already been employed, on two base sequences included in each of the combinations, under a condition in which partial sequences to be compared for the two base sequences include 3'-ends of the two base sequences, to determine local alignment scores.
Further, if the steps (c-2) and (d-2) and the steps (e-2) and (f-2) are performed in parallel, the step (e-2) is preferably replaced with step (e'-2) below.
(e'-2) A second global alignment step of performing pairwise global alignment, for all combinations for selecting base sequences of two candidate primers and all combinations for selecting a base sequence of one candidate primer and a base sequence of one primer that has already been employed from among base sequences of candidate primers for PCR amplifying a second target region having a priority of 2 among base sequences of candidate primers generated in the plurality-of-candidate-primer-base-sequence generation step and from among base sequences of primers that have already been employed, on base sequences having a preset sequence length and including 3'-ends of two base sequences included in each of the combinations, to determine global alignment scores.

Further, when the at least one candidate amplification region includes three or more candidate amplification regions, when three or more target regions are selected in the plurality-of-target-region selection step, when base sequences of candidate primers for PCR amplifying each of the three or more target regions are generated in the plurality-of-candidate-primer-base-sequence generation step, and when base sequences of primers for PCR amplifying third and subsequent target regions having the third and subsequent highest priorities are employed, the steps from the second local alignment step to the second primer employment step are repeated for the third and subsequent target regions.

<Description of Steps>

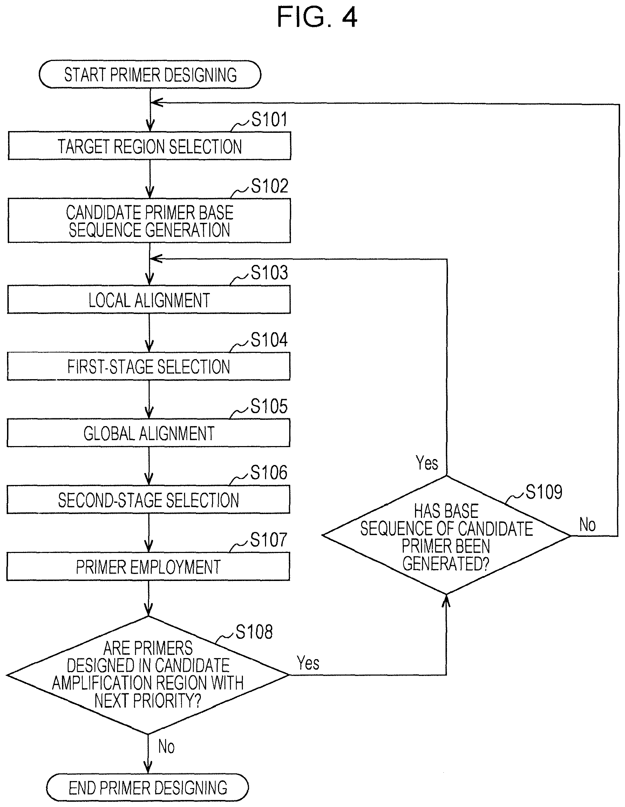

The steps in the first to third aspects of the primer design method after priority setting will be described with reference to Fig. 4 to Fig. 6, if necessary.

«Target Region Selection Step»

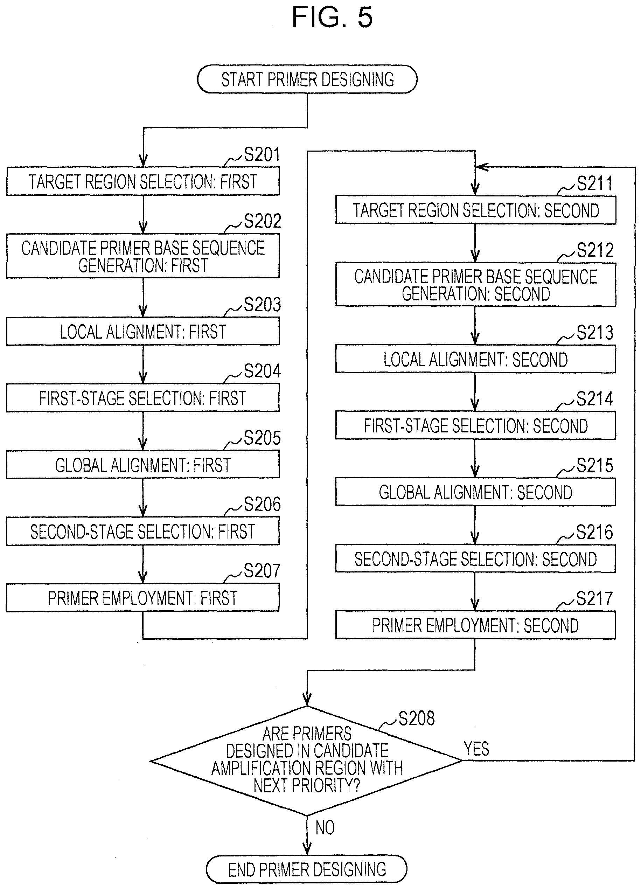

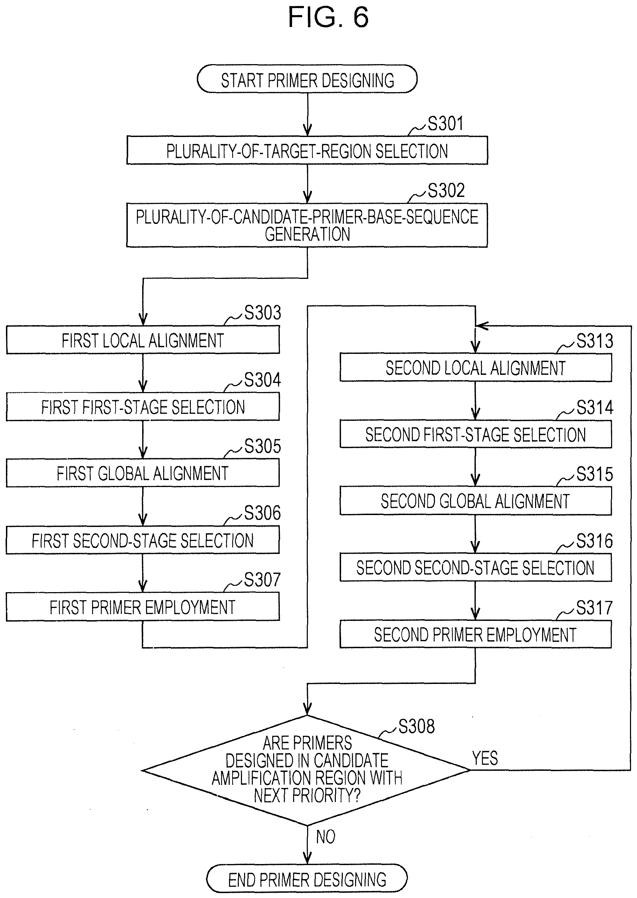

As used herein, target region selection step S101 (Fig. 4), first step of target region selection S201 and second step of target region selection S211 (Fig. 5), and plurality-of-target-region selection step S301 (Fig. 6) are collectively referred to sometimes simply as "target region selection step".

(First aspect: target region selection step S101)

In Fig. 4, this step is represented as "target region selection".

In the first aspect, the target region selection step (a) is a step of selecting a target region from candidate amplification regions with set priorities in order of priority.

(Second aspect: first step of target region selection S201 and second step of target region selection S211)

In Fig. 5, these steps are represented as "target region selection: first" and "target region selection: second".

In the second aspect, the first step of target region selection ($a_1$) is a step of selecting a candidate amplification region having the highest priority as a first target region from among the candidate amplification regions with set priorities, and the second step of target region selection ($a_2$) is a step of selecting, as a second target region, a candidate amplification region having the highest priority from among candidate amplification regions that have not been selected among candidate amplification regions with set priorities.

In the second aspect, candidate amplification regions are selected one by one in order of priority.

(Third aspect: plurality-of-target-region selection step S301)

In Fig. 6, this step is represented as "plurality-of-target-region selection".

In the third aspect, the plurality-of-target-region selection step (a-0) is a step of selecting a plurality of target regions from candidate amplification regions with set priorities in order from highest to lowest in terms of priority.

In the third aspect, a plurality of candidate amplification regions are selected in order of priority. Preferably, all the candidate amplification regions with set priorities are selected.

«Candidate Primer Base Sequence Generation Step»

Candidate primer base sequence generation step S102 (Fig. 4), first step of candidate primer base sequence generation S202 and second step of candidate primer base sequence generation S212 (Fig. 5), and plurality-of-candidate-primer-base-sequence generation step S302 (Fig. 6) are collectively referred to sometimes simply as "candidate primer base sequence generation step".

(First aspect: candidate primer base sequence generation step S102)

In Fig. 4, this step is represented as "candidate primer base sequence generation".

In the first aspect, the candidate primer base sequence generation step (b) is a step of generating at least one base sequence of a candidate primer for PCR amplifying a target region on the basis of each of base sequences of respective neighboring regions located at two ends of the target region on genomic DNA.

(Second aspect: first step of candidate primer base sequence generation S202 and second step of candidate primer base sequence generation S212)

In Fig. 5, these steps are represented as "candidate primer base sequence generation: first" and "candidate primer base sequence generation: second".

In the second aspect, the first step of candidate primer base sequence generation ($b_1$) is a step of generating at least one base sequence of a candidate primer for PCR amplifying a first target region on the basis of each of base sequences of respective neighboring regions located at two ends of the first target region on genomic DNA, and the second step of candidate primer base sequence generation ($b_2$) is a step of generating at least one base sequence of a candidate primer for PCR amplifying a second target region on the basis of each of base sequences of respective neighboring regions located at two ends of the second target region on genomic DNA.

In the second aspect, the generation of a base sequence of a candidate primer, the selection of a candidate primer, and the employment of a primer are performed for one target region, and similar steps are repeated for the next target region.

(Third aspect: plurality-of-candidate-primer-base-sequence generation step S302)

In Fig. 6, this step is represented as "plurality-of-candidate-primer-base-sequence generation".

In the third aspect, the plurality-of-candidate-primer-base-sequence generation step (b-0) generates at least one base sequence of a candidate primer for PCR amplifying each of a plurality of target regions on the basis of each of base sequences of respective neighboring regions located at two ends of each of the plurality of target regions on genomic DNA.

In the third aspect, base sequences of candidate primers are generated for all the plurality of target regions, and selection and employment are repeated in the subsequent steps.

(Neighboring region)

Respective neighboring regions located at two ends of a target region are collectively referred to as regions outside the 5'-end of the target region and regions outside the 3'-end of the target region. The area inside the target region is not included in the neighboring regions.

The length of a neighboring region is not specifically limited, and is preferably less than or equal to a length that allows extension of a neighboring region by PCR, and more preferably less than or equal to the upper limit of the length of the DNA fragment to be amplified. In particular, the length of a neighboring region is preferably a length that facilitates application of concentration selection and/or sequence reading. The length of a neighboring region may be changed as appropriate in accordance with the type or the like of enzyme (DNA polymerase) to be used in PCR. The specific length of a neighboring region is preferably about 20 to 500 bases, more preferably about 20 to 300 bases, even more preferably about 20 to 200 bases, and still more preferably about 50 to 200 bases.

(Primer design parameter)

In addition, to generate a base sequence of a candidate primer, careful attention is required to the same points as those in a common method for designing primers, such as primer length, GC content (corresponding to the total mole percentage of guanine (G) and cytosine (C) in all nucleic acid bases), melting temperature (temperature at which 50% of double-stranded DNA is dissociated into single-stranded DNA, referred to sometimes as "Tm value", from Melting Temperature, in "°C"), and sequence deviation.

• Primer Length

The primer length (number of nucleotides) is not specifically limited, and is preferably 15-mer to 45-mer, more preferably 20-mer to 45-mer, and even more preferably 20-mer to 30-mer. A primer length in this range facilitates the designing of a primer excellent in specificity and amplification efficiency.

• Primer GC Content

The primer GC content is not specifically limited, and is preferably 40 mol% to 60 mol%, and more preferably 45 mol% to 55 mol%. A GC content in this range is less likely to cause a problem of a reduction in specificity and amplification efficiency due to a high-order structure.

• Primer Tm Value

The primer Tm value is not specifically limited, and is preferably in a range of 50°C to 65°C, and more preferably in a range of 55°C to 65°C.

In a primer pair and a primer set, the difference between the Tm values of primers is set to preferably 5°C or less, and more preferably 3°C or less.

The Tm value can be calculated using software such as OLIGO Primer Analysis Software (manufactured by Molecular Biology Insights Inc.) or Primer3 (http://www-genome.wi.mit.edu/ftp/distribution/software/).

Alternatively, the Tm value can be calculated in accordance with the formula below based on the numbers of A's, T's, G's, and C's (represented as nA, nT, nG, and nC, respectively) in a base sequence of a primer.

$$\text{Tm value (°C)} = 2(nA + nT) + 4(nC + nG)$$

The method for calculating the Tm value is not limited to those described above, and the Tm value can be calculated using any of various well-known methods.

• Base Deviation of Primer

A base sequence of a candidate primer is preferably a sequence having entirely no deviation of bases. For example, it is desirable to avoid a partially GC-rich sequence and a partially AT-rich sequence.

It is also desirable to avoid consecutive T's and/or C's (polypyrimidine) and consecutive A's and/or G's (polypurine).

• 3'-end of Primer

For the 3'-end base sequence, furthermore, it is preferable to avoid a GC-rich sequence or an AT-rich sequence. The base at the 3'-end is preferably, but is not limited to, G or C.

«Specificity Check Step»

A specificity check step may be performed (not illustrated) to evaluate the specificity of a base sequence of a candidate primer on the basis of the sequence complementarity of a base sequence of each candidate primer, which is generated in the "candidate primer base sequence generation step", to chromosomal DNA.

A specificity check may be performed in the following manner. Local alignment is performed between a base sequence of chromosomal DNA and a base sequence of a candidate primer, and it can be evaluated that the base sequence of the candidate primer has low complementarity to the genomic DNA and has high specificity when the local alignment score is less than a preset value. It is desirable to perform local alignment also on complementary strands of the chromosomal DNA. This is because whereas a primer is single-stranded DNA, chromosomal DNA is double-stranded. Alternatively, instead of a base sequence of a candidate primer, a base sequence complementary thereto may be used.

In addition, homology search may be performed against a genomic DNA base sequence database by using a base sequence of a candidate primer as a query sequence. Examples of a homology search tool include BLAST (Basic Local Alignment Search Tool) (Altschul, S. A., four others, "Basic Local Alignment Search Tool", Journal of Molecular Biology, October 1990, Vol. 215, pp. 403-410) and FASTA (Pearson, W. R., one other, "Improved tools for biological sequence comparison", Proceedings of the National Academy of Sciences of the United States of America, the National Academy of Sciences of the United States of America, April 1988, Vol. 85, pp. 2444-2448). As a result of homology search, local alignment can be obtained.

Threshold values for scores and local alignment scores are not specifically limited and may be set as appropriate in accordance with the length of a base sequence of a candidate primer and/or PCR conditions or the like. When a homology search tool is used, specified values for the homology search tool may be used.

For example, as the score, match (complementary base) = +1, mismatch (non-complementary base) = -1, and indel (insertion and/or deletion) = -3 may be employed, and the threshold value may be set to +15.

If a base sequence of a candidate primer has complementarity to a base sequence at an unexpected position on chromosomal DNA and has low specificity, an artifact, rather than a target region, may be amplified in PCR performed using a primer of the base sequence, and the artifact is thus removed.

«Local Alignment Step»

As used herein, local alignment step S103 (Fig. 4), first step of local alignment S203 and second step of local alignment S213 (Fig. 5), and first local alignment step S303 and second local alignment step S313 (Fig. 6) are collectively referred to sometimes simply as "local alignment step".

(First aspect: local alignment step S103)

In Fig. 4, this step is represented as "local alignment".
In the first aspect, the local alignment step (c) is a step of performing pairwise local alignment, for all combinations for selecting base sequences of two candidate primers from among base sequences of candidate primers generated in the candidate primer base sequence generation step, on two base sequences included in each of the combinations, under a condition in which partial sequences to be compared for the two base sequences include 3'-ends of the two base sequences, to determine local alignment scores.

(Second aspect: first step of local alignment S203 and second step of local alignment S213)

In Fig. 5, these steps are represented as "local alignment: first" and "local alignment: second".
In the second aspect, the first step of local alignment ($c_1$) is a step of performing pairwise local alignment, for all combinations for selecting base sequences of two candidate primers from among base sequences of candidate primers generated in the first step of candidate primer base sequence generation, on two base sequences included in each of the combinations, under a condition in which partial sequences to be compared for the two base sequences include 3'-ends of the two base sequences, to determine local alignment scores, and the second step of local alignment ($c_2$) is a step of performing pairwise local alignment, for all combinations for selecting base sequences of two candidate primers and all combinations for selecting a base sequence of one candidate primer and a base sequence of one primer that has already been employed from among base sequences of candidate primers generated in the second step of candidate primer base sequence generation and from among base sequences of primers that have already been employed, on two base sequences included in each of the combinations, under a condition in which partial sequences to be compared for the two base sequences include 3'-ends of the two base sequences, to determine local alignment scores.

(Third aspect: first local alignment step S303 and second local alignment step S313)

In Fig. 6, these steps are represented as "first local alignment" and "second local alignment".
In the third aspect, the first local alignment step (c-1) is a step of performing pairwise local alignment, for all combinations for selecting base sequences of two candidate primers from among base sequences of candidate primers for PCR amplifying a first target region having the highest priority among base sequences of candidate primers generated in the plurality-of-candidate-primer-base-sequence generation step, on two base sequences included in each of the combinations, under a condition in which partial sequences to be compared for the two base sequences include 3'-ends of the two base sequences, to determine local alignment scores, and the second local alignment step (c-2) is a step of performing pairwise local alignment, for all combinations for selecting base sequences of two candidate primers and all combinations for selecting a base sequence of one candidate primer and a base sequence of one primer that has already been employed from among base sequences of candidate primers for PCR amplifying a second target region having a priority of 2 among base sequences of candidate primers generated in the plurality-of-candidate-primer-base-sequence generation step and from among base sequences of primers that have already been employed, on two base sequences included in each of the combinations, under a condition in which partial sequences to be compared for the two base sequences include 3'-ends of the two base sequences, to determine local alignment scores.

(Method for local alignment)

A combination of base sequences to be subjected to local alignment may be a combination selected with allowed overlap or a combination selected without allowed overlap. However, if the probability of primer dimer formation between primers having the same base sequence has not yet been evaluated, it is preferable to use a combination selected with allowed overlap.
The total number of combinations is given by "$_pH_2 = {}_{p+1}C_2 = (p + 1)!/2(p - 1)!$" when combinations are selected with allowed overlap, and is given by "$_pC_2 = p(p - 1)/2$" when combinations are selected without allowed overlap, where p denotes the total number of base sequences to be subjected to local alignment.
Local alignment is alignment to be performed on partial sequences and allows local examination of high complementarity fragments.

In the present invention, however, unlike typical local alignment performed on base sequences, local alignment is performed under the condition that "partial sequences to be compared include the 3'-ends of the base sequences", so that partial sequences to be compared include the 3'-ends of both the base sequences.

In the present invention, furthermore, in a preferred aspect, local alignment is performed under the condition that "partial sequences to be compared include the 3'-ends of the base sequences", that is, the condition that "partial sequences to be compared take into account only alignment that starts at the 3'-end of one of the sequences and ends at the 3'-end of the other sequence", so that partial sequences to be compared include the 3'-ends of both the base sequences.

Note that in local alignment, a gap may be inserted. The gap refers to an insertion and/or deletion (indel) of a base.

In local alignment, furthermore, a match is determined when bases in a base sequence pair are complementary to each other, and a mismatch is determined when bases in a base sequence pair are not complementary to each other.

Alignment is performed such that a score is set for each of a match, a mismatch, and an indel and the total score is maximum. The scores may be set as appropriate. For example, scores may be set as in Table 1 below. In Table 1, "-" indicates a gap (insertion and/or deletion (indel)).

Table 1

|   | A | T | G | c |   |
|---|---|---|---|---|---|
| A | -1 | +1 : | -1 | -1 | -1 |
| T | +1 | -1 : | -1 | -1 | -1 |
| G | -1 | -1 | -1 | +1 | -1 |
| C | -1 | -1 | +1 | -1 | -1 |
| - | -1 | -1 : | -1 | -1 |   |

"-":gap(indel)

For example, consideration is given to local alignment of base sequences with SEQ ID NOs: 1 and 2 given in Table 2 below. Here, scores are assumed to be given in Table 1.

Table 2

| | Base sequence (5' → 3') |
|---|---|
| SEQ ID NO: 1 | TAGCCGGATGTGGGAGATGG |
| SEQ ID NO: 2 | CCAGCATTGGAAAGATCTGG |

A dot matrix given in Table 3 is generated from the base sequences with SEQ ID NOs: 1 and 2. Specifically, the base sequence with SEQ ID NO: 1 is arranged from left to right in a 5' to 3' direction, and the base sequence with SEQ ID NO: 2 is arranged from bottom to top in a 5' to 3' direction, with grids of complementary bases filled with "●" to obtain a dot matrix given in Table 3.

Table 3

|  | T | A | G | C | C | G | G | A | T | G | T | G | G | G | A | G | A | T | G | G |
|---|---|---|---|---|---|---|---|---|---|---|---|---|---|---|---|---|---|---|---|---|
| G |  |  |  | ● | ● |  |  |  |  |  |  |  |  |  |  |  |  |  |  |  |
| G |  |  |  | ● | ● |  |  |  |  |  |  |  |  |  |  |  |  |  |  |  |
| T |  | ● |  |  |  |  |  | ● |  |  |  |  |  |  | ● |  | ● |  |  |  |
| C |  |  | ● |  |  | ● | ● |  |  | ● |  | ● | ● | ● |  | ● |  |  | ● | ● |
| T |  | ● |  |  |  |  |  |  | ● |  |  |  |  |  | ● |  | ● |  |  |  |
| A | ● |  |  |  |  |  |  |  | ● |  | ● |  |  |  |  |  |  | ● |  |  |
| G |  |  |  | ● | ● |  |  |  |  |  |  |  |  |  |  |  |  |  |  |  |
| A | ● |  |  |  |  |  |  |  | ● |  | ● |  |  |  |  |  |  | ● |  |  |
| A | ● |  |  |  |  |  |  |  | ● |  | ● |  |  |  |  |  |  | ● |  |  |
| A | ● |  |  |  |  |  |  |  | ● |  | ● |  |  |  |  |  |  | ● |  |  |
| G |  |  |  | ● | ● |  |  |  |  |  |  |  |  |  |  |  |  |  |  |  |
| G |  |  |  | ● | ● |  |  |  |  |  |  |  |  |  |  |  |  |  |  |  |
| T |  | ● |  |  |  |  |  | ● |  |  |  |  |  |  | ● |  | ● |  |  |  |
| T |  | ● |  |  |  |  |  | ● |  |  |  |  |  |  | ● |  | ● |  |  |  |
| A | ● |  |  |  |  |  |  |  | ● |  | ● |  |  |  |  |  |  | ● |  |  |
| C |  |  | ● |  |  | ● | ● |  |  | ● |  | ● | ● | ● |  | ● |  |  | ● | ● |
| G |  |  |  | ● | ● |  |  |  |  |  |  |  |  |  |  |  |  |  |  |  |
| A | ● |  |  |  |  |  |  |  | ● |  | ● |  |  |  |  |  |  | ● |  |  |
| C |  |  | ● |  |  | ● | ● |  |  | ● |  | ● | ● | ● |  | ● |  |  | ● | ● |
| C |  |  | ● |  |  | ● | ● |  |  | ● |  | ● | ● | ● |  | ● |  |  | ● | ● |

SEQ ID NO: 1→ (columns), SEQ ID NO: 2→ (rows)

The dot matrix given in Table 3 yields alignment of partial sequences (pairwise alignment) as given in Table 4 below (see a portion indicated by the diagonal line in Table 3). In Table 4, a match is denoted by "|" and a mismatch is denoted by ":".

Table 4

| Partial sequence from SEQ ID NO: 1 | 5'-  C  C  G  G  A  T  G  T  G  G  G  A  G  A  T  G  G  -3' |
|---|---|
| Partial sequence from SEQ ID NO: 2 |     \|  \|  :  \|  \|  \|  :  \|  :  :  :  :  :  \|  \|  \|  :<br>3'-  G  G  T  C  T  A  G  A  A  A  G  G  T  T  A  C  G  -5' |

This (pairwise) alignment includes nine matches, eight mismatches, and no indel (gap).

Thus, the local alignment score based on this (pairwise) alignment is given by $(+1) \times 9 + (-1) \times 8 + (-1) \times 0 = +1$.

Note that the alignment (pairwise alignment) may be obtained using, instead of the dot matrix method exemplified herein, the dynamic programming method, the word method, or any of various other methods.

«First-Stage Selection Step»

As used herein, first-stage selection step S104 (Fig. 4), first step of first-stage selection S204 and second step of first-stage selection S214 (Fig. 5), and first first-stage selection step S304 and second first-stage selection step S314 (Fig. 6) are collectively referred to sometimes simply as "first-stage selection step".

(First aspect: first-stage selection step S104)

In Fig. 4, this step is represented as "first-stage selection".

In the first aspect, the first-stage selection step (d) is a step of performing first-stage selection of base sequences of candidate primers for PCR amplifying the target region on the basis of the local alignment scores.

(Second aspect: first step of first-stage selection S204 and second step of first-stage selection S214)

In Fig. 5, these steps are represented as "first-stage selection: first" and "first-stage selection: second".
In the second aspect, the first step of first-stage selection ($d_1$) is a step of performing first-stage selection of base sequences of candidate primers for PCR amplifying the first target region on the basis of the local alignment scores, and the second step of first-stage selection ($d_2$) is a step of performing first-stage selection of base sequences of candidate primers for PCR amplifying the second target region on the basis of the local alignment scores.

(Third aspect: first first-stage selection step S304 and second first-stage selection step S314)

In Fig. 6, these steps are represented as "first first-stage selection" and "second first-stage selection".
In the third aspect, the first first-stage selection step (d-1) is a step of performing first-stage selection of base sequences of candidate primers for PCR amplifying the first target region on the basis of the local alignment scores, and the second first-stage selection step (d-2) is a step of performing first-stage selection of base sequences of candidate primers for PCR amplifying the second target region on the basis of the local alignment scores.

(Method for first-stage selection)

A threshold value for local alignment scores (referred to also as "first threshold value") is set in advance.
If a local alignment score is less than the first threshold value, the combination of two base sequences is determined to have low probability of dimer formation, and then the subsequent step is performed.
On the other hand, if a local alignment score is not less than the first threshold value, the combination of two base sequences is determined to have high probability of primer dimer formation, and no further steps are performed for the combination.
The first threshold value is not specifically limited and can be set as appropriate. For example, the first threshold value may be set in accordance with PCR conditions such as the amount of genomic DNA that is a template for polymerase chain reaction.
Here, consideration is given to a case where the first threshold value is set to "+3" in the example provided in the "local alignment" described above.
In the above example, the local alignment score is "+1" and is less than the first threshold value, that is, "+3". Thus, the combination of the base sequences with SEQ ID NOs: 1 and 2 can be determined to have low probability of primer dimer formation.
Note that this step is performed on all the combinations for which local alignment scores are calculated in the local alignment step S103, the first step of local alignment S203, the second step of local alignment S213, the first local alignment step S303, or the second local alignment step S313.

«Global Alignment Step»

As used herein, global alignment step S105 (Fig. 4), first step of global alignment S205 and second step of global alignment S215 (Fig. 5), and first global alignment step S305 and second global alignment step S315 (Fig. 6) are collectively referred to sometimes simply as "global alignment step".

(First aspect: global alignment step S105)

In Fig. 4, this step is represented as "global alignment".
In the first aspect, the global alignment step (e) is a step of performing pairwise global alignment, for all combinations for selecting base sequences of two candidate primers from among base sequences of candidate primers selected in the first-stage selection step, on base sequences having a preset sequence length and including 3'-ends of two base sequences included in each of the combinations, to determine global alignment scores.

(Second aspect: first step of global alignment S205 and second step of global alignment S215)

In Fig. 5, these steps are represented as "global alignment: first" and "global alignment: second".
In the second aspect, the first step of global alignment ($e_1$) is a step of performing pairwise global alignment, for all combinations for selecting base sequences of two candidate primers from among base sequences of candidate primers selected in the first step of first-stage selection, on base sequences having a preset sequence length and including 3'-ends of two base sequences included in each of the combinations, to determine global alignment scores, and the second step of global alignment ($e_2$) is a step of performing pairwise global alignment, for all combinations for selecting base sequences of two candidate primers and all combinations for selecting a base sequence of one candidate primer and a base sequence of one primer that has already been employed from among base sequences of candidate primers selected in the second step of first-stage selection and from among base sequences of primers that have already been employed, on base sequences having a preset sequence length and including 3'-ends of two base sequences included in each of the combinations, to determine global alignment scores.

(Third aspect: first global alignment step S305 and second global alignment step S315)

In Fig. 6, these steps are represented as "first global alignment" and "second global alignment".

In the third aspect, the first global alignment step (e-1) is a step of performing pairwise global alignment, for all combinations for selecting base sequences of two candidate primers from among base sequences of candidate primers selected in the first first-stage selection step, on base sequences having a preset sequence length and including 3'-ends of two base sequences included in each of the combinations, to determine global alignment scores, and the second global alignment step (e-2) is a step of performing pairwise global alignment, for all combinations for selecting base sequences of two candidate primers and all combinations for selecting a base sequence of one candidate primer and a base sequence of one primer that has already been employed from among base sequences of candidate primers selected in the second first-stage selection step and from among base sequences of primers that have already been employed, on base sequences having a preset sequence length and including 3'-ends of two base sequences included in each of the combinations, to determine global alignment scores.

(Method for global alignment)

A global alignment score is determined by extracting two primers from the group consisting of all the candidate primers generated in the "candidate primer base sequence generation step" (when the "local alignment step" and the "first-stage selection step" are performed previously, if there is a combination of candidate primers having local alignment scores less than the first threshold value, all the candidate primers included in the combination) and all the primers that have already been employed (only when there is present a primer that has already been employed) and by performing pairwise global alignment on base sequences having a preset sequence length and including the 3'-ends of the extracted primers.

A combination of base sequences to be subjected to global alignment may be a combination selected with allowed overlap or a combination selected without allowed overlap. However, if the probability of primer dimer formation between primers having the same base sequence has not yet been evaluated, it is preferable to use a combination selected with allowed overlap.

The total number of combinations is given by "$_xH_2 = _{x+1}C_2 = (x + 1)!/2(x - 1)!$" when combinations are selected with allowed overlap, and is given by "$_xC_2 = x(x - 1)/2$" when combinations are selected without allowed overlap, where x denotes the total number of base sequences to be subjected to global alignment.

Global alignment is alignment to be performed on "entire sequences" and allows examination of the complementarity of the entire sequences.

As used here, the "entire sequence" refers to the entire base sequence having a preset sequence length and including the 3'-end of a base sequence of a candidate primer.

Note that in global alignment, a gap may be inserted. The gap refers to an insertion and/or deletion (indel) of a base.

In global alignment, furthermore, a match is determined when bases in a base sequence pair are complementary to each other, and a mismatch is determined when bases in a base sequence pair are not complementary to each other.

Alignment is performed such that a score is set for each of a match, a mismatch, and an indel and the total score is maximum. The scores may be set as appropriate. For example, scores may be set as in Table 1 above. In Table 1, "-" indicates a gap (insertion and/or deletion (indel)).

For example, consideration is given to global alignment of, for base sequences with SEQ ID NOs: 1 and 2 given in Table 5 below, three bases (indicated by capital letters) at the 3'-end of each base sequence. Here, scores are assumed to be given in Table 1.

| Table 5 | |
| --- | --- |
| | Base sequence (5' → 3') |
| SEQ ID NO: 1 | tagccggatgtgggagaTGG |

(continued)

| | Base sequence (5' → 3') |
|---|---|
| SEQ ID NO: 2 | ccagcattggaaagatcTGG |

Global alignment is performed on three bases (indicated by capital letters) at the 3'-end of the base sequence with SEQ ID NO: 1 and the base sequence of three bases (indicated by capital letters) at the 3'-end of SEQ ID NO: 2 so as to obtain a maximum score, yielding alignment (pairwise alignment) given in Table 6 below. In Table 6, a mismatch is denoted by ":".

Table 6

| Three bases at 3'-end of SEQ ID NO: 1 | 5'- | T | G | G | -3' |
|---|---|---|---|---|---|
| Three bases at 3'-end of SEQ ID NO: 2 | | : | : | : | |
| | 3'- | G | G | T | -5' |

This (pairwise) alignment includes 3 mismatches and no match and indel (gap).

Thus, the global alignment score based on this (pairwise) alignment is given by $(+1) \times 0 + (-1) \times 3 + (-1) \times 0 = -3$.

Note that alignment (pairwise alignment) may be obtained using the dot matrix method, the dynamic programming method, the word method, or any of various other methods. «Second-Stage Selection Step»

As used herein, second-stage selection step S106 (Fig. 4), first step of second-stage selection S206 and second step of second-stage selection S216 (Fig. 5), and first second-stage selection step S306 and second second-stage selection step S316 (Fig. 6) are collectively referred to sometimes simply as "second-stage selection step".

(First aspect: second-stage selection step S106)

In Fig. 4, this step is represented as "second-stage selection".

In the first aspect, the second-stage selection step (f) is a step of performing second-stage selection of base sequences of candidate primers for PCR amplifying the target region on the basis of the global alignment scores.

(Second aspect: first step of second-stage selection S206 and second step of second-stage selection S216)

In Fig. 5, these steps are represented as "second-stage selection: first" and "second-stage selection: second".

In the second aspect, the first step of second-stage selection $(f_1)$ is a step of performing second-stage selection of base sequences of candidate primers for PCR amplifying the first target region on the basis of the global alignment scores, and the second step of second-stage selection $(f_2)$ is a step of performing second-stage selection of base sequences of candidate primers for PCR amplifying the second target region on the basis of the global alignment scores.

(Third aspect: first second-stage selection step S306 and second second-stage selection step S316)

In Fig. 6, these steps are represented as "first second-stage selection" and "second second-stage selection".

In the third aspect, the first second-stage selection step (f-1) is a step of performing second-stage selection of base sequences of candidate primers for PCR amplifying the first target region on the basis of the global alignment scores, and the second second-stage selection step (f-2) is a step of performing second-stage selection of base sequences of candidate primers for PCR amplifying the second target region on the basis of the global alignment scores.

(Method for second-stage selection)

A threshold value for global alignment scores (referred to also as "second threshold value") is set in advance.

If a global alignment score is less than the second threshold value, the combination of two base sequences is determined to have low probability of dimer formation, and then the subsequent step is performed.

On the other hand, if a global alignment score is not less than the second threshold value, the combination of two base sequences is determined to have high probability of dimer formation, and no further steps are performed for the combination.

The second threshold value is not specifically limited and can be set as appropriate. For example, the second threshold value may be set in accordance with PCR conditions such as the amount of genomic DNA that is a template for polymerase chain reaction.

Note that base sequences including several bases from the 3'-ends of primers are set to be the same, whereby a global alignment score determined by performing pairwise global alignment on base sequences having a preset number of bases including the 3'-ends of the base sequences of the respective primers can be made less than the second threshold value.

Here, consideration is given to a case where the second threshold value is set to "+3" in the example provided in the "global alignment step" described above.

In the above example, the global alignment score is "-3" and is less than the second threshold value, that is, "+3". Thus, the combination of the base sequences with SEQ ID NOs: 1 and 2 can be determined to have low probability of primer dimer formation.

Note that this step is performed on all the combinations for which global alignment scores are calculated in the global alignment step S105, the first step of global alignment S205, the second step of global alignment S215, the first global alignment step S305, or the second global alignment step S315.

In addition, to reduce the amount of computation, preferably, both the "global alignment step" and the "second-stage selection step" are performed previously, and both the "local alignment step" and the "first-stage selection step" are performed on a combination of base sequences of primers that have been subjected to the "second-stage selection step". In particular, as the number of target regions and the number of base sequences of candidate primers increase, the effect of reducing the amount of computation increases, leading to an increase in the speed of the overall processing.

This is because in the "global alignment step", global alignment is performed on base sequences having a short length, that is, the "preset sequence length", which requires less computation than the calculation of a local alignment score to find partial sequences having high complementarity from the entire base sequences under the condition that the 3'-ends are included, resulting in higher-speed processing. Note that it is known that a commonly known algorithm allows global alignment to be performed at a higher speed than local alignment when the alignments are performed on sequences having the same length.

«Amplification Sequence Length Check Step»

A combination of base sequences of candidate primers determined to have low probability of primer dimer formation in the "first-stage selection step" and the "second-stage selection step" may be subjected to an amplification sequence length check step (not illustrated) to compute the distance between the ends of the base sequences of the candidate primers on the chromosomal DNA to determine whether the distance falls within a preset range.

If the distance between the ends of the base sequences falls within the preset range, the combination of the base sequences of the candidate primers can be determined to be likely to amplify the target region in a suitable manner. The distance between the ends of the base sequences of the candidate primers is not specifically limited and may be set as appropriate in accordance with PCR conditions such as the type of enzyme (DNA polymerase). For example, the range may be set to any of various ranges such as a range of 100 to 200 bases (pairs), a range of 120 to 180 bases (pairs), a range of 140 to 180 bases (pairs), a range of 140 to 160 bases (pairs), and a range of 160 to 180 bases (pairs).

«Primer Employment Step»

As used herein, primer employment step S107 (Fig. 4), first step of primer employment S207 and second step of primer employment S217 (Fig. 5), and first primer employment step S307 and second primer employment step S317 (Fig. 6) are collectively referred to sometimes simply as "primer employment step".

(First aspect: primer employment step S107)

In Fig. 4, this step is represented as "primer employment".

In the first aspect, the primer employment step (g) is a step of employing, as base sequences of primers for PCR amplifying the target region, base sequences of candidate primers selected in both the first-stage selection step and the second-stage selection step.

(Second aspect: first step of primer employment S207 and second step of primer employment S217)

In Fig. 5, these steps are represented as "primer employment: first" and "primer employment: second".

In the second aspect, the first step of primer employment ($g_1$) is a step of employing, as base sequences of primers for PCR amplifying the first target region, base sequences of candidate primers selected in both the first step of first-stage selection and the first step of second-stage selection, and the second step of primer employment ($g_2$) is a step of employing, as base sequences of primers for PCR amplifying the second target region, base sequences of candidate primers selected in both the second step of first-stage selection and the second step of second-stage selection.

(Third aspect: first primer employment step S307 and second primer employment step S317)

In Fig. 6, these steps are represented as "first primer employment" and "second primer employment".
In the third aspect, the first primer employment step (g-1) is a step of employing base sequences of candidate primers selected in both the first first-stage selection step and the first second-stage selection step as base sequences of primers for PCR amplifying the first target region, and the second primer employment step (g-2) is a step of employing base sequences of candidate primers selected in both the second first-stage selection step and the second second-stage selection step as base sequences of primers for PCR amplifying the second target region.

(Method for primer employment)

In the primer employment step, base sequences of candidate primers having a local alignment score less than the first threshold value, where the local alignment score is determined by performing pairwise local alignment on base sequences of candidate primers under the condition that the partial sequences to be compared include the 3'-ends of the base sequences, and having a global alignment score less than the second threshold value, where the global alignment score is determined by performing pairwise global alignment on base sequences having a preset number of bases including the 3'-ends of the base sequences of the candidate primers, are employed as base sequences of primers for amplifying a target region.
For example, consideration is given to the employment of base sequences with SEQ ID NOs: 1 and 2 given in Table 7 as base sequences of primers for amplifying a target region.

Table 7

| | Base sequence (5' → 3') |
|---|---|
| SEQ ID NO: 1 | TAGCCGGATGTGGGAGATGG |
| SEQ ID NO: 2 | CCAGCATTGGAAAGATCTGG |

As described previously, for the combination of SEQ ID NO: 1 and SEQ ID NO: 2, the local alignment score is "+1" and is thus less than the first threshold value, that is, "+3". Further, the global alignment score is "-3" and is thus less than the second threshold value, that is, "+3".
Accordingly, the base sequence of the candidate primer indicated by SEQ ID NO: 1 and the base sequence of the candidate primer indicated by SEQ ID NO: 2 can be employed as base sequences of primers for amplifying a target region.

«Primer Design for Other Candidate Amplification Regions»

After the employment of primers for one candidate amplification region, primers may further be designed in the candidate amplification region having the next priority.
In the first aspect, if base sequences of candidate primers for a candidate amplification region having the next priority have been generated in the candidate primer base sequence generation step S102, the local alignment step S103 and the following steps are performed. If base sequences of candidate primers for a candidate amplification region having the next priority have not been generated, a candidate amplification region having the next priority is not selected in the target region selection step S101. Thus, in the target region selection step S101, a candidate amplification region having the next priority is selected. Then, in the candidate primer base sequence generation step S102, base sequences of candidate primers for the candidate amplification region are generated. After that, the local alignment step S103 and the subsequent steps are performed.
In the second aspect, the process repeats from the second step of target region selection S211.
In the third aspect, base sequences of candidate primers for the candidate amplification regions selected in the plurality-of-target-region selection step S301 have been generated in the plurality-of-candidate-primer-base-sequence generation step S302. Thus, the process repeats from the second local alignment step S313.

«Feature Point in Designing of Primers, etc.»

In brief, a feature point in the designing of primers, etc. after candidate amplification regions are assigned priorities is that a plurality of specific target regions are selected, nearby base sequences are searched for, the complementarity of the found nearby base sequences to each of extracted primer sets is examined, and base sequences with low complementarity are selected to obtain a primer group in which primers are not complementary to each other and for which a target region is included in an object to be amplified.

A feature point in the examination of the complementarity of base sequences of primers is to generate a primer group so as to reduce the complementarity of the entire sequences by using local alignment and reduce the complementarity of ends of the base sequences of the primers by using global alignment.

[Examples]

[Example 1]

Primers for multiplex PCR for PCR amplifying candidate amplification regions given in Table 8 are designed.

This Example aims to design primers for 53 or more of 85 candidate amplification regions.

Table 8

| | | | Candidate amplification region | | | | | | | | |
|---|---|---|---|---|---|---|---|---|---|---|---|
| No. | SNP name | Chromosome | Coordinate | No. | SNP name | Chromosome | Coordinate | No. | SNP name | Chromosome | Coordinate |
| 1 | V01 | 13 | 20763642 | 31 | V31 | 13 | 36857639 | 61 | V61 | 13 | 103397937 |
| 2 | V02 | 13 | 21562948 | 32 | V32 | 13 | 36886469 | 62 | V62 | 13 | 103410782 |
| 3 | V03 | 13 | 23905711 | 33 | V33 | 13 | 39264690 | 63 | V63 | 13 | 103410914 |
| 4 | V04 | 13 | 23909162 | 34 | V34 | 13 | 39265512 | 64 | V64 | 13 | 103718308 |
| 5 | V05 | 13 | 24471039 | 35 | V35 | 13 | 40261945 | 65 | V65 | 13 | 109318370 |
| 6 | V06 | 13 | 24797913 | 36 | V36 | 13 | 41767338 | 66 | V66 | 13 | 109550367 |
| 7 | V07 | 13 | 24798120 | 37 | V37 | 13 | 41834744 | 67 | V67 | 13 | 109779906 |
| 8 | V08 | 13 | 24890157 | 38 | V38 | 13 | 42032572 | 68 | V68 | 13 | 109831944 |
| 9 | V09 | 13 | 24890228 | 39 | V39 | 13 | 46067593 | 69 | V69 | 13 | 111098226 |
| 10 | V10 | 13 | 24895393 | 40 | V40 | 13 | 46946157 | 70 | V70 | 13 | 111156499 |
| 11 | V11 | 13 | 24895437 | 41 | V41 | 13 | 47469940 | 71 | V71 | 13 | 111298392 |
| 12 | V12 | 13 | 24895559 | 42 | V42 | 13 | 51417535 | 72 | V72 | 13 | 111368164 |
| 13 | V13 | 13 | 25265103 | 43 | V43 | 13 | 52515354 | 73 | V73 | 13 | 111870037 |
| 14 | V14 | 13 | 25487103 | 44 | V44 | 13 | 52544805 | 74 | V74 | 13 | 111938511 |
| 151 | V15 | 13 | 25670919 | 45 | V45 | 13 | 53286950 | 75 | V75 | 13 | 113052388 |
| 16 | V16 | 13 | 25670984 | 46 | V46 | 13 | 53608479 | 76 | V76 | 13 | 113333684 |
| 17 | V17 | 13 | 25671008 | 47 | V47 | 13 | 67800935 | 77 | V77 | 13 | 113536132 |
| 18 | V18 | 13 | 25671062 | 48 | V48 | 13 | 67802339 | 78 | V78 | 13 | 113720476 |
| 19 | V19 | 13 | 25671080 | 49 | V49 | 13 | 76427253 | 79 | V79 | 13 | 113728781 |
| 20 | V20 | 13 | 27845654 | 50 | V50 | 13 | 77738664 | 80 | V80 | 13 | 113801737 |
| 21 | V21 | 13 | 28610183 | 51 | V51 | 13 | 80911525 | 81 | V81 | 13 | 113818817 |
| 22 | V22 | 13 | 30107067 | 52 | V52 | 13 | 92345579 | 82 | V82 | 13 | 113826090 |
| 23 | V23 | 13 | 31821240 | 53 | V53 | 13 | 95858978 | 83 | V83 | 13 | 113897320 |
| 24 | V24 | 13 | 32885654 | 54 | V54 | 13 | 97639414 | 84 | V84 | 13 | 114309226 |

| Candidate amplification region | | | | | | | | | | | |
|---|---|---|---|---|---|---|---|---|---|---|---|
| No. | SNP name | Chromosome | Coordinate | No. | SNP name | Chromosome | Coordinate | No. | SNP name | Chromosome | Coordinate |
| 25 | V25 | 13 | 32929232 | 55 | V55 | 13 | 99537217 | 85 | V85 | 13 | 114524944 |
| 26 | V26 | 13 | 36385031 | 56 | V56 | 13 | 101795422 | | | | |
| 27 | V27 | 13 | 36402426 | 57 | V57 | 13 | 102366825 | | | | |
| 28 | V28 | 13 | 36743177 | 58 | V58 | 13 | 103275386 | | | | |
| 29 | V29 | 13 | 36744910 | 59 | V59 | 13 | 103339365 | | | | |
| 30 | V30 | 13 | 36801415 | 60 | V60 | 13 | 103396716 | | | | |

(Setting of first priorities)

First priorities were set for candidate amplification regions V1 to V85 given in Table 8 in order of coordinate value.

(Primer design after setting of first priorities)

As a result of designing primers for PCR amplifying the candidate amplification regions according to the first priorities, primers were successfully designed in the following 52 candidate amplification regions.

Table 9

| No. | Name | Chromosome | SNP coordinate | No. | Name | Chromosome | SNP coordinate |
|---|---|---|---|---|---|---|---|
| 1 | V01 | 13 | 20763642 | 24 | V41 | 13 | 47469940 |
| 2 | V04 | 13 | 23909162 | 25 | V42 | 13 | 51417535 |
| 3 | V05 | 13 | 24471039 | 26 | V43 | 13 | 52515354 |
| 4 | V06 | 13 | 24797913 | 27 | V44 | 13 | 52544805 |
| 5 | V08 | 13 | 24890157 | 28 | V47 | 13 | 67800935 |
| 6 | V12 | 13 | 24895559 | 29 | V52 | 13 | 92345579 |
| 7 | V13 | 13 | 25265103 | 30 | V53 | 13 | 95858978 |
| 8 | V16 | 13 | 25670984 | 31 | V54 | 13 | 97639414 |
| 9 | V20 | 13 | 27845654 | 32 | V55 | 13 | 99537217 |
|  |  |  |  | 33 | V57 | 13 | 102366825 |
|  |  |  |  | 34 | V58 | 13 | 103275386 |
|  |  |  |  | 35 | V60 | 13 | 103396716 |
| 10 | V21 | 13 | 28610183 | 36 | V61 | 13 | 103397937 |
| 11 | V22 | 13 | 30107067 | 37 | V64 | 13 | 103718308 |
| 12 | V23 | 13 | 31821240 | 38 | V65 | 13 | 109318370 |
| 13 | V24 | 13 | 32885654 | 39 | V67 | 13 | 109779906 |
| 14 | V26 | 13 | 36385031 | 40 | V68 | 13 | 109831944 |
| 15 | V28 | 13 | 36743177 | 41 | V69 | 13 | 111098226 |
| 16 | V29 | 13 | 36744910 | 42 | V70 | 13 | 111156499 |
| 17 | V30 | 13 | 36801415 | 43 | V71 | 13 | 111298392 |
| 18 | V32 | 13 | 36886469 | 44 | V74 | 13 | 111938511 |
| 19 | V33 | 13 | 39264690 | 45 | V75 | 13 | 113052388 |
| 20 | V35 | 13 | 40261945 | 46 | V77 | 13 | 113536132 |
| 21 | V36 | 13 | 41767338 | 47 | V78 | 13 | 113720476 |
| 22 | V37 | 13 | 41834744 | 48 | V79 | 13 | 113728781 |
| 23 | V40 | 13 | 46946157 | 49 | V80 | 13 | 113801737 |
|  |  |  |  | 50 | V81 | 13 | 113818817 |
|  |  |  |  | 51 | V83 | 13 | 113897320 |
|  |  |  |  | 52 | V84 | 13 | 114309226 |

(Setting of second priorities)

The candidate amplification regions V1 to V85 were segmented into four blocks each including 20 candidate amplification regions and one block including five candidate amplification regions in order of coordinate value, that is, block 1 including the candidate amplification regions V1 to V20, block 2 including the candidate amplification regions V21 to V40, block 3 including the candidate amplification regions V41 to V60, block 4 including the candidate amplification regions V61 to V80, and block 5 including the candidate amplification regions V81 to V85.

Then, random permutation was performed within each block to obtain a sequence in which the order of the candidate amplification regions within the block was randomly changed.

The blocks 1 to 5 were joined together in this order, and block segmentation was canceled to obtain a sequence in which the candidate amplification regions V1 to V85 were rearranged.

The order of V1 to V85 was set as the order of second priorities.

(Primer design after setting of second priorities)

As a result of designing primers for PCR amplifying the candidate amplification regions according to the second priorities, primers were successfully designed in the following 54 candidate amplification regions.

Table 10

| Candidate amplification region | | | | | | | |
|---|---|---|---|---|---|---|---|
| No. | Name | Chromosome | SNP coordinate | No. | Name | Chromosome | SNP coordinate |
| 1 | V13 | 13 | 25265103 | 24 | V53 | 13 | 95858978 |
| 2 | V05 | 13 | 24471039 | 25 | V48 | 13 | 67802339 |
| 3 | V08 | 13 | 24890157 | 26 | V46 | 13 | 53608479 |
| 4 | V06 | 13 | 24797913 | 27 | V57 | 13 | 102366825 |
| 5 | V16 | 13 | 25670984 | 28 | V52 | 13 | 92345579 |
| 6 | V12 | 13 | 24895559 | 29 | V60 | 13 | 103396716 |
| 7 | V20 | 13 | 27845654 | 30 | V55 | 13 | 99537217 |
| 8 | V04 | 13 | 23909162 | 31 | V44 | 13 | 52544805 |
| 9 | V01 | 13 | 20763642 | 32 | V41 | 13 | 47469940 |
| | | | | 33 | V58 | 13 | 103275386 |
| | | | | 34 | V47 | 13 | 67800935 |
| | | | | 35 | V43 | 13 | 52515354 |
| | | | | 36 | V42 | 13 | 51417535 |
| | | | | 37 | V54 | 13 | 97639414 |
| 10 | V33 | 13 | 39264690 | 38 | V73 | 13 | 111870037 |
| 11 | V28 | 13 | 36743177 | 39 | V65 | 13 | 109318370 |
| 12 | V30 | 13 | 36801415 | 40 | V68 | 13 | 109831944 |
| 13 | V26 | 13 | 36385031 | 41 | V77 | 13 | 113536132 |
| 14 | V36 | 13 | 41767338 | 42 | V80 | 13 | 113801737 |
| 15 | V37 | 13 | 41834744 | 43 | V75 | 13 | 113052388 |
| 16 | V32 | 13 | 36886469 | 44 | V64 | 13 | 103718308 |
| 17 | V40 | 13 | 46946157 | 45 | V61 | 13 | 103397937 |
| 18 | V35 | 13 | 40261945 | 46 | V78 | 13 | 113720476 |
| 19 | V24 | 13 | 32885654 | 47 | V67 | 13 | 109779906 |
| 20 | V21 | 13 | 28610183 | 48 | V79 | 13 | 113728781 |

(continued)

| Candidate amplification region | | | | | | | |
|---|---|---|---|---|---|---|---|
| No. | Name | Chromosome | SNP coordinate | No. | Name | Chromosome | SNP coordinate |
| 21 | V23 | 13 | 31821240 | 49 | V69 | 13 | 111098226 |
| 22 | V29 | 13 | 36744910 | 50 | V71 | 13 | 111298392 |
| 23 | V22 | 13 | 30107067 | 51 | V74 | 13 | 111938511 |
| | | | | 52 | V84 | 13 | 114309226 |
| | | | | 53 | V81 | 13 | 113818817 |
| | | | | 54 | V83 | 13 | 113897320 |

Reference Signs List

11    arithmetic means (CPU)
12    storage means (memory)
13    auxiliary storage means (storage)
14    input means (keyboard)
15    auxiliary input means (mouse)
16    display means (monitor)
17    output means (printer)

**Claims**

1.  A method for designing primers for multiplex PCR, for amplifying t or more candidate amplification regions among n candidate amplification regions on a genome, comprising:

    a first priority setting step of assigning first priorities from 1 through n to n candidate amplification regions on genomic DNA;
    a first primer design step of designing primers for PCR amplifying the candidate amplification regions sequentially in order of the first priorities, starting from a candidate amplification region that is highest of the first priorities;
    a first success/failure determination step of determining that designing of primers is complete when $m \geq t$ is satisfied, where m denotes the number of candidate amplification regions in which primers are successfully designed in the first primer design step, and determining that a subsequent step is performed when $m < t$ is satisfied;
    a second priority setting step of assigning second priorities from 1 through n to the n candidate amplification regions, the second priorities being in different order than the first priorities; and
    a second primer design step of designing primers for PCR amplifying the candidate amplification regions sequentially in order of the second priorities, starting from a candidate amplification region that is highest of the second priorities,
    the second priority setting step including the steps of:

        inputting identification information and first priority information of the n candidate amplification regions via input means and storing the identification information and the first priority information in storage means;

by arithmetic means, arranging the n candidate amplification regions in order of the first priorities to generate a first sequence including the n candidate amplification regions as elements, and storing the first sequence in the storage means;

by the arithmetic means, segmenting the n candidate amplification regions arranged in order of the first priorities into j blocks so that an i-th block includes $k_i$ candidate amplification regions, and storing the j blocks in the storage means;

by the arithmetic means, rearranging, within at least one block among the j blocks, the candidate amplification regions included in the at least one block, and storing the rearranged candidate amplification regions in the storage means; and

by the arithmetic means, sequentially joining first through j-th blocks together to cancel block segmentation to generate a second sequence, an order of the n candidate amplification regions included in the second sequence being set as an order of the second priorities of the n candidate amplification regions,

where n is an integer satisfying $n \geq 4$, t is an integer satisfying $2 \leq t \leq n$, m is an integer satisfying $0 \leq m \leq n$, i is an integer satisfying $1 \leq i \leq j$, j is an integer satisfying $2 \leq j \leq n/2$, and $k_i$ is an integer satisfying $2 \leq k_i \leq \{n - 2 \times (j - 1)\}$.

2. The method for designing primers for multiplex PCR according to claim 1, further comprising, after the second primer design step,

a second success/failure determination step of determining that designing of primers is complete when $m' \geq t$ is satisfied, where m' denotes the number of candidate amplification regions in which primers are successfully designed in the second primer design step, and determining that the second priority setting step is performed again when $m' < t$ is satisfied,

where m' is an integer satisfying $0 \leq m' \leq n$.

3. The method for designing primers for multiplex PCR according to claim 1 or 2, wherein in the second priority setting step, an order of the candidate amplification regions within the at least one block is changed randomly.

# FIG. 1

| ARITHMETIC MEANS (CPU) ~11 | STORAGE MEANS (MEMORY) ~12 | AUXILIARY STORAGE MEANS (STORAGE) ~13 |

| INPUT MEANS (KEYBOARD) ~14 | AUXILIARY INPUT MEANS (MOUSE) ~15 | DISPLAY MEANS (MONITOR) ~16 | OUTPUT MEANS (PRINTER) ~17 |

# FIG. 2

START

↓

FIRST PRIORITY SETTING STEP ~S11

↓

FIRST PRIMER DESIGN STEP ~S12

↓

NUMBER m OF CANDIDATE AMPLIFICATION REGIONS IN WHICH PRIMERS ARE SUCCESSFULLY DESIGNED ~S13

< t →

≥ t

SECOND PRIORITY SETTING STEP ~S21

↓

SECOND PRIMER DESIGN STEP ~S22

↓

NUMBER m' OF CANDIDATE AMPLIFICATION REGIONS IN WHICH PRIMERS ARE SUCCESSFULLY DESIGNED ~S23

< t

≥ t

END

# FIG. 3

(a) CANDIDATE AMPLIFICATION REGION $X_1$ $X_2$ $X_3$ $X_4$ $X_5$ $X_6$ $X_7$ $X_8$ $X_9$

⇩ ARRANGE REGIONS IN ORDER OF FIRST PRIORITIES

(b) FIRST SEQUENCE (FROM LEFT TO RIGHT) $X_1$ $X_2$ $X_3$ $X_4$ $X_5$ $X_6$ $X_7$ $X_8$ $X_9$

⇩ BLOCK SEGMENTATION

BLOCK 1　　　　BLOCK 2　　　　BLOCK 3

(c) MAINTAIN ORIGINAL ORDER $X_1$ $X_2$ $X_3$ | $X_4$ $X_5$ $X_6$ | $X_7$ $X_8$ $X_9$

⇩ REARRANGE REGIONS WITHIN BLOCKS

BLOCK 1　　　　BLOCK 2　　　　BLOCK 3

(d) CHANGE ORIGINAL ORDER $X_2$ $X_3$ $X_1$ | $X_5$ $X_6$ $X_4$ | $X_8$ $X_9$ $X_7$

⇩ CANCEL BLOCK SEGMENTATION/ REJOIN BLOCKS
REGIONS ARE ARRANGED IN ORDER OF SECOND PRIORITIES

(e) SECOND SEQUENCE (FROM LEFT TO RIGHT) $X_2$ $X_3$ $X_1$ $X_5$ $X_6$ $X_4$ $X_8$ $X_9$ $X_7$

# FIG. 4

START PRIMER DESIGNING

~S101
TARGET REGION SELECTION

~S102
CANDIDATE PRIMER BASE
SEQUENCE GENERATION

~S103
LOCAL ALIGNMENT

~S104
FIRST-STAGE SELECTION

~S105
GLOBAL ALIGNMENT

~S106
SECOND-STAGE SELECTION

~S107
PRIMER EMPLOYMENT

~S108
ARE PRIMERS
DESIGNED IN CANDIDATE
AMPLIFICATION REGION WITH
NEXT PRIORITY?

Yes

No

~S109
HAS BASE
SEQUENCE OF CANDIDATE
PRIMER BEEN
GENERATED?

Yes

No

END PRIMER DESIGNING

# FIG. 5

```
                    ( START PRIMER DESIGNING )
                              │
          ┌───────────────────┴──────────────────────┐
          │                                           │
          ▼                    ～S201                  ▼                        ～S211
┌────────────────────────────┐            ┌────────────────────────────────┐
│ TARGET REGION SELECTION: FIRST │        │ TARGET REGION SELECTION: SECOND │
└────────────────────────────┘            └────────────────────────────────┘
          │                    ～S202                  │                        ～S212
          ▼                                           ▼
┌────────────────────────────┐            ┌────────────────────────────────┐
│ CANDIDATE PRIMER BASE SEQUENCE │        │ CANDIDATE PRIMER BASE SEQUENCE  │
│      GENERATION: FIRST        │          │      GENERATION: SECOND         │
└────────────────────────────┘            └────────────────────────────────┘
          │                    ～S203                  │                        ～S213
          ▼                                           ▼
┌────────────────────────────┐            ┌────────────────────────────────┐
│    LOCAL ALIGNMENT: FIRST    │          │    LOCAL ALIGNMENT: SECOND     │
└────────────────────────────┘            └────────────────────────────────┘
          │                    ～S204                  │                        ～S214
          ▼                                           ▼
┌────────────────────────────┐            ┌────────────────────────────────┐
│  FIRST-STAGE SELECTION: FIRST │        │ FIRST-STAGE SELECTION: SECOND  │
└────────────────────────────┘            └────────────────────────────────┘
          │                    ～S205                  │                        ～S215
          ▼                                           ▼
┌────────────────────────────┐            ┌────────────────────────────────┐
│   GLOBAL ALIGNMENT: FIRST    │          │   GLOBAL ALIGNMENT: SECOND     │
└────────────────────────────┘            └────────────────────────────────┘
          │                    ～S206                  │                        ～S216
          ▼                                           ▼
┌────────────────────────────┐            ┌────────────────────────────────┐
│ SECOND-STAGE SELECTION: FIRST │        │ SECOND-STAGE SELECTION: SECOND │
└────────────────────────────┘            └────────────────────────────────┘
          │                    ～S207                  │                        ～S217
          ▼                                           ▼
┌────────────────────────────┐            ┌────────────────────────────────┐
│   PRIMER EMPLOYMENT: FIRST   │          │   PRIMER EMPLOYMENT: SECOND    │
└────────────────────────────┘            └────────────────────────────────┘
```

ARE PRIMERS DESIGNED IN CANDIDATE AMPLIFICATION REGION WITH NEXT PRIORITY?  ～S208

YES

NO

( END PRIMER DESIGNING )

# FIG. 6

```
        ( START PRIMER DESIGNING )
                    |
                    v                        ~S301
        +-----------------------------------------+
        | PLURALITY-OF-TARGET-REGION SELECTION    |
        +-----------------------------------------+
                    |
                    v                        ~S302
        +-----------------------------------------+
        | PLURALITY-OF-CANDIDATE-PRIMER-BASE-      |
        |            SEQUENCE GENERATION           |
        +-----------------------------------------+
                    |
```

| ~S303 | ~S313 |
|---|---|
| FIRST LOCAL ALIGNMENT | SECOND LOCAL ALIGNMENT |
| ~S304 | ~S314 |
| FIRST FIRST-STAGE SELECTION | SECOND FIRST-STAGE SELECTION |
| ~S305 | ~S315 |
| FIRST GLOBAL ALIGNMENT | SECOND GLOBAL ALIGNMENT |
| ~S306 | ~S316 |
| FIRST SECOND-STAGE SELECTION | SECOND SECOND-STAGE SELECTION |
| ~S307 | ~S317 |
| FIRST PRIMER EMPLOYMENT | SECOND PRIMER EMPLOYMENT |

```
                    |
                    v              ~S308
              / ARE PRIMERS \
             /  DESIGNED IN   \
            <  CANDIDATE       >------ YES
             \ AMPLIFICATION  /
              \ REGION WITH  /
               \NEXT PRIORITY?/
                    |
                    | NO
                    v
        ( END PRIMER DESIGNING )
```

## INTERNATIONAL SEARCH REPORT

| International application No. |
| --- |
| PCT/JP2017/032287 |

**A. CLASSIFICATION OF SUBJECT MATTER**
C12N15/09(2006.01)i, C12Q1/68(2006.01)i, G06F19/20(2011.01)i

According to International Patent Classification (IPC) or to both national classification and IPC

**B. FIELDS SEARCHED**

Minimum documentation searched (classification system followed by classification symbols)
C12N15/09, C12Q1/68, G06F19/20

Documentation searched other than minimum documentation to the extent that such documents are included in the fields searched
Published examined utility model applications of Japan      1922-1996
Registered utility model specifications of Japan      1996-2017
Published unexamined utility model applications of Japan      1971-2017
Published registered utility model applications of Japan      1994-2017

Electronic data base consulted during the international search (name of data base and, where practicable, search terms used)
JSTPlus/JMEDPlus/JST7580 (JDreamlII), CAplus/MEDLINE/BIOSIS/wPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2008/004691 A1 (SHIMATSU CORPORATION) 10 January 2008, claims, embodiments, & US 2010/0070452 A1, claims, example & CN 101484898 A | 1-3 |
| A | JP 2015-136314 A (K.K. DNAFORM) 30 July 2015, claims (Family: none) | 1-3 |
| A | JP 2000-308487 A (JAPAN SCIENCE AND TECHNOLOGY CORP.) 07 November 2000, claims, paragraphs [0035]-[0045], fig. 5 (Family: none) | 1-3 |

☒ Further documents are listed in the continuation of Box C.  ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| | |

| Name and mailing address of the ISA/ Japan Patent Office 3-4-3, Kasumigaseki, Chiyoda-ku, Tokyo 100-8915, Japan | Authorized officer Telephone No. |
| --- | --- |

Form PCT/ISA/210 (second sheet) (January 2015)

**INTERNATIONAL SEARCH REPORT**

International application No.

PCT/JP2017/032287

C (Continuation).   DOCUMENTS CONSIDERED TO BE RELEVANT

| Category* | Citation of document, with indication, where appropriate, of the relevant passages | Relevant to claim No. |
|---|---|---|
| A | CHEN, Y. et al., Design of mutiplex PCR primers using heuristic algorithm for sequential deletion applications, Computational Biology and Chemistry, 2009, Vol. 33, pp. 181-188, especially Abstract, pp. 182-184, ISSN: 1476-9271 | 1-3 |

Form PCT/ISA/210 (continuation of second sheet) (January 2015)

**REFERENCES CITED IN THE DESCRIPTION**

**Non-patent literature cited in the description**

- **ALTSCHUL, S. A.** Basic Local Alignment Search Tool. *Journal of Molecular Biology,* October 1990, vol. 215, 403-410

- Improved tools for biological sequence comparison. **PEARSON, W. R.** Proceedings of the National Academy of Sciences of the United States of America. National Academy of Sciences of the United States of America, April 1988, vol. 85, 2444-2448